# EUROPEAN PATENT APPLICATION

(11) **EP 2 319 866 A2**
(43) Date of publication of application: **11.05.2011**
(21) Application number: 10185649.0
(22) Date of filing: 22.08.2003
(51) Int. Cl.: C07K 14/47, C12Q 1/68, G01N 33/50, G01N 33/53, A61K 38/17, C07K 16/18, A01K 67/027, C12N 15/12, A61K 48/00

(54) **Polymorphisms in the human genes for OCT1 and their use in diagnostic and therapeutic applications**

(30) Priority: 23.08.2002 EP 02018904
(62) Divisional of application: 03792418.0
(71) Applicant: PGXHealth, LLC, Newton, MA 02458 (US)
(72) Inventor: Kerb, Reinhold, 81241, München (DE); Koepsell, Hermann, 97204, Höchberg (DE); Brinkmann, Ulrich, 82362, Weilheim (DE)
(74) Representative: Dörries, Hans Ulrich

(57) **Abstract**

The present invention relates to a polymorphic OCT1 polynucleotide. Moreover, the invention relates to genes or vectors comprising the polynucleotides of the invention and to a host cell genetically engineered with the polynucleotide or gene of the invention. Further, the invention relates to methods for producing molecular variant polypeptides or fragments thereof, methods for producing cells capable of expressing a molecular variant polypeptide and to a polypeptide or fragment thereof encoded by the polynucleotide or the gene of the invention or which is obtainable by the method or from the cells produced by the method of the invention. Furthermore, the invention relates to an antibody which binds specifically the polypeptide of the invention. Moreover, the invention relates to a transgenic non-human animal. The invention also relates to a solid support comprising one or a plurality or the above mentioned polynucleotides, genes, vectors, polypeptides, antibodies or host cells. Furthermore, methods of identifying a polymorphism, identifying and obtaining a pro-drug or drug or an inhibitor are also encompassed by the present invention. In addition, the invention relates to methods for producing of a pharmaceutical composition and to methods of diagnosing a disease. Further, the invention relates to a method of detection of the polynucleotide of the invention. Furthermore, comprised by the present invention are a diagnostic and a pharmaceutical composition. Even more, the invention relates to uses of the polynucleotides, genes, vectors, polypeptides or antibodies of the invention. Finally, the invention relates to a diagnostic kit.

SEQUENCE LISTING

<110>  PGxHealth, LLC

<120>  Polymorphisms in the human gene for OCT1 and their use in
diagnostic and therapeutic applications

<130>  G2224PCT

<160>  67

<170>  PatentIn version 3.1

<210>  1
<211>  19
<212>  DNA
<213>  Homo sapiens

<400>  1
aaatggccaa ttgaattca                                                    19

<210>  2
<211>  19
<212>  DNA
<213>  Homo sapiens

<400>  2
taccctttca ccagcatgt                                                    19

<210>  3
<211>  19
<212>  DNA
<213>  Homo sapiens

<400>  3
gcatgtcagc ctgctgagc                                                    19

<210>  4
<211>  19
<212>  DNA
<213>  Homo sapiens

<400>  4
ctgagccagt gctgtggct                                                    19

<210>  5
<211>  21
<212>  DNA
<213>  Homo sapiens

<400>  5
ccttggccag cgcaggcgct a                                                 21

<210>  6
<211>  19

<212>  DNA
<213>  Homo sapiens

<400>  6
tcccacctgt cctccatgt                                                    19


<210>  7
<211>  19
<212>  DNA
<213>  Homo sapiens

<400>  7
tctccctcct tcctagatg                                                    19


<210>  8
<211>  19
<212>  DNA
<213>  Homo sapiens

<400>  8
gaccgcgtga gccgcatct                                                    19


<210>  9
<211>  19
<212>  DNA
<213>  Homo sapiens

<400>  9
tgttggcggc ggcagcctg                                                    19


<210>  10
<211>  18
<212>  DNA
<213>  Homo sapiens

<400>  10
tgcctcgtca tttttatc                                                     18


<210>  11
<211>  19
<212>  DNA
<213>  Homo sapiens

<400>  11
tcccaggcag tcgaagtgt                                                    19


<210>  12
<211>  19
<212>  DNA
<213>  Homo sapiens

<400>  12
catcatttct caggcaatc                                                    19

<210>  13
<211>  19
<212>  DNA
<213>  Homo sapiens

<400>  13
ggtgagtgcg tggaacagg                                                    19


<210>  14
<211>  19
<212>  DNA
<213>  Homo sapiens

<400>  14
aggaacctca gagtgatgg                                                    19


<210>  15
<211>  19
<212>  DNA
<213>  Homo sapiens

<400>  15
attggctgta ctctaatgg                                                    19


<210>  16
<211>  19
<212>  DNA
<213>  Homo sapiens

<400>  16
ccttcttttt cagctcggc                                                    19


<210>  17
<211>  19
<212>  DNA
<213>  Homo sapiens

<400>  17
tgaagcggta ttgggcctg                                                    19


<210>  18
<211>  19
<212>  DNA
<213>  Homo sapiens

<400>  18
cacacatggg tctgtgctt                                                    19


<210>  19
<211>  19
<212>  DNA
<213>  Homo sapiens

<400>  19
tgaccagtta gaattaact                                                    19


<210>  20
<211>  19
<212>  DNA
<213>  Homo sapiens

<400>  20
agccccaaca tggggaggg                                                    19


<210>  21
<211>  17
<212>  DNA
<213>  Homo sapiens

<400>  21
tggtaagttg tctgctt                                                      17


<210>  22
<211>  19
<212>  DNA
<213>  Homo sapiens

<400>  22
ctgccagagc cctggggtg                                                    19


<210>  23
<211>  21
<212>  DNA
<213>  Homo sapiens

<400>  23
cgggcttctt gtttggctct c                                                 21


<210>  24
<211>  21
<212>  DNA
<213>  Homo sapiens

<400>  24
ccccatggcc gtgtcaaatt t                                                 21


<210>  25
<211>  19
<212>  DNA
<213>  Homo sapiens

<400>  25
ccaccagctg taatagtcc                                                    19


<210>  26
<211>  21

<212>  DNA
<213>  Homo sapiens

<400>  26
ttttgcagct tggcagtggg c                                                21


<210>  27
<211>  21
<212>  DNA
<213>  Homo sapiens

<400>  27
ttaactccaa tttttaattt t                                                21


<210>  28
<211>  11
<212>  PRT
<213>  Homo sapiens

<400>  28

Ala Glu Leu Ser Gln Cys Cys Gly Trp Ser Pro
1               5                   10


<210>  29
<211>  11
<212>  PRT
<213>  Homo sapiens

<400>  29

Ala Phe Leu Gly Gln Arg Arg Arg Tyr Glu Val
1               5                   10


<210>  30
<211>  11
<212>  PRT
<213>  Homo sapiens

<400>  30

Thr Ile Asp Arg Val Ser Arg Ile Tyr Pro Met
1               5                   10


<210>  31
<211>  11
<212>  PRT
<213>  Homo sapiens

<400>  31

Ser Asn Leu Leu Ala Ala Ala Ala Cys Leu Val
1               5                   10

<210>  32
<211>  11
<212>  PRT
<213>  Homo sapiens

<400>  32

Ala Ala Cys Leu Val Ile Phe Ile Ser Pro Asp
1               5                   10


<210>  33
<211>  11
<212>  PRT
<213>  Homo sapiens

<400>  33

Phe Val Arg Asn Leu Arg Val Met Val Cys Ser
1               5                   10


<210>  34
<211>  11
<212>  PRT
<213>  Homo sapiens

<400>  34

Leu Asn Ala Gly Phe Leu Phe Gly Ser Leu Gly
1               5                   10


<210>  35
<211>  11
<212>  PRT
<213>  Homo sapiens

<400>  35

Ile Tyr Pro Met Ala Val Ser Asn Leu Leu Ala
1               5                   10


<210>  36
<211>  23
<212>  DNA
<213>  Homo sapiens

<400>  36
aacgatttga tcagatggcc acg                                               23


<210>  37
<211>  19
<212>  DNA
<213>  Homo sapiens

<400>  37
ccagacaccc acgaactgc                                                    19

<210>  38
<211>  21
<212>  DNA
<213>  Homo sapiens

<400>  38
aaacagccca gggataccga g                                                21


<210>  39
<211>  21
<212>  DNA
<213>  Homo sapiens

<400>  39
cccacagtat cccaaagcag g                                                21


<210>  40
<211>  19
<212>  DNA
<213>  Homo sapiens

<400>  40
ctccgactgt gacccttgg                                                   19


<210>  41
<211>  20
<212>  DNA
<213>  Homo sapiens

<400>  41
aactggtgcc ccgcaagctc                                                  20


<210>  42
<211>  19
<212>  DNA
<213>  Homo sapiens

<400>  42
ccgagcttct gaacgcacg                                                   19


<210>  43
<211>  19
<212>  DNA
<213>  Homo sapiens

<400>  43
actggtccct cgagaggac                                                   19


<210>  44
<211>  22
<212>  DNA
<213>  Homo sapiens

<400>  44
atcctcttga gggattacag cc                                              22


<210>  45
<211>  19
<212>  DNA
<213>  Homo sapiens

<400>  45
ccccagacga atctgcacc                                                  19


<210>  46
<211>  20
<212>  DNA
<213>  Homo sapiens

<400>  46
atgggtgtga agcacggtgg                                                 20


<210>  47
<211>  28
<212>  DNA
<213>  Homo sapiens

<400>  47
gagtattcca ctgtctctaa tctatagc                                        28


<210>  48
<211>  24
<212>  DNA
<213>  Homo sapiens

<400>  48
tttcttcagt ctctgactca tgcc                                            24


<210>  49
<211>  28
<212>  DNA
<213>  Homo sapiens

<400>  49
aaaaaacttt gtagacaaag gtagcacc                                        28


<210>  50
<211>  28
<212>  DNA
<213>  Homo sapiens

<400>  50
aaaagttaga taagacaaac ttccaggc                                        28


<210>  51

<211>  22
<212>  DNA
<213>  Homo sapiens

<400>  51
ggctgcatct ttaggaagca cc                                                22


<210>  52
<211>  24
<212>  DNA
<213>  Homo sapiens

<400>  52
aagctgcagg tattggcatt gtac                                              24


<210>  53
<211>  22
<212>  DNA
<213>  Homo sapiens

<400>  53
agccagaaga catcccaaga gc                                                22


<210>  54
<211>  29
<212>  DNA
<213>  Homo sapiens

<400>  54
aatgaaggca atgtttcctt tacgtactc                                         29


<210>  55
<211>  29
<212>  DNA
<213>  Homo sapiens

<400>  55
aagacataca aatatctgta aagctctcc                                         29


<210>  56
<211>  24
<212>  DNA
<213>  Homo sapiens

<400>  56
aaacaggcta taagctcgaa tggg                                              24


<210>  57
<211>  22
<212>  DNA
<213>  Homo sapiens

<400>  57
cctagatcga atgcacaggt gg                                                22

<210> 58
<211> 25
<212> DNA
<213> Homo sapiens

<400> 58
tacacacaca caaatgaaga ggtgg 25


<210> 59
<211> 27
<212> DNA
<213> Homo sapiens

<400> 59
tggtttgaaa tcagtttgct gtccaag 27


<210> 60
<211> 21
<212> DNA
<213> Homo sapiens

<400> 60
ggccctacca aactgcaaag c 21


<210> 61
<211> 24
<212> DNA
<213> Homo sapiens

<400> 61
atggtatgag gcaagtattg ggtg 24


<210> 62
<211> 25
<212> DNA
<213> Homo sapiens

<400> 62
cttactcacc tcacgtgtag atctg 25


<210> 63
<211> 30
<212> DNA
<213> Homo sapiens

<400> 63
cttaagtatg actttgctaa ataggctgtc 30


<210> 64
<211> 22
<212> DNA
<213> Homo sapiens

<400>  64
cttcccttct tgtgtcagta gc                                                22

<210>  65
<211>  29
<212>  DNA
<213>  Homo sapiens

<400>  65
gagctaccat tataacatgt agtgatgac                                         29

<210>  66
<211>  20
<212>  DNA
<213>  Homo sapiens

<400>  66
cctctccctt tcgatgctcc                                                   20

<210>  67
<211>  27
<212>  DNA
<213>  Homo sapiens

<400>  67
   caaggttttc ttgaagcact tacatgc                                            27

## Description

### Technical Field

The present invention relates to a polymorphic OCT1 polynucleotide. Moreover, the invention relates to genes or vectors comprising the polynucleotides of the invention and to a host cell genetically engineered with the polynucleotide or gene of the invention. Further, the invention relates to methods for producing molecular variant polypeptides or fragments thereof, methods for producing cells capable of expressing a molecular variant polypeptide and to a polypeptide or fragment thereof encoded by the polynucleotide or the gene of the invention or which is obtainable by the method or from the cells produced by the method of the invention. Furthermore, the invention relates to an antibody which binds specifically the polypeptide of the invention. Moreover, the invention relates to a transgenic non-human animal. The invention also relates to a solid support comprising one or a plurality of the above mentioned polynucleotides, genes, vectors, polypeptides, antibodies or host cells. Furthermore, methods of identifying a polymorphism, identifying and obtaining a pro-drug or drug or an inhibitor are also encompassed by the present invention. In addition, the invention relates to methods for producing of a pharmaceutical composition and to methods of diagnosing a disease. Further, the invention relates to a method of detection of the polynucleotide of the invention. Furthermore, comprised by the present invention are a diagnostic and a pharmaceutical composition. Even more, the invention relates to uses of the polynucleotides, genes, vectors, polypeptides or antibodies of the invention. Finally, the invention relates to a diagnostic kit.

Several documents are cited throughout the text of this specification. Each of the documents cited herein (including any manufacturer's specification, instructions etc.) and references therein are hereby incorporated by reference.

### Background of the invention

The OCT-family comprises a subfamily of electrogenic transporters, that translocates a variety of organic cations and contains the subtypes OCT1, OCT2 and OCT3 (Dresser, J. Pharm. Sci. 90 (2001), 397-421; Koepsell, J. Membr. Biol. 167 (1999), 103-117). The human OCT1 (hOCT1, gene SLC22A1) is predicted to have 12 transmembrane domains (TMDs) (Gorboulev, DNA Cell Biol. 16 (1997), 871-881) and contains one large, extracellularly localized, hydrophilic loop between TMD1/2 (Meyer-Wentrup, Biochem. Biophys. Res. Commun. 248 (1998), 673-678). OCT1 is most strongly expressed at the sinusoidal membrane of hepatocytes (Meyer-Wentrup, Biochem. Biophys. Res. Commun. 248 (1998), 673-678) and at lower levels in epithelial cells and neurons of the intestine, in the placenta, in the kidney and in the heart (Arndt, Am. J. Physiol. Renal. Physiol. 281 (2001), F454-468; Chen, J. Neurosci. 21 (2001), 6348-6361; Wessler, Br. J. Pharmacol. 134 (2001), 951-956). OCT1 translocates a broad array of organic cations with various structures and molecular weights including endogenous compounds such as choline, guanidine, dopamine, serotonin, histamine, acetylcholine, norepinephrine, creatinine, and the prostaglandins E₂ and F_{2α}, as well as exogeneous compounds such as tetraethylammonium (TEA), 1-methyl-4-phenylpyridinium (MPP), N-methylquinine, and N-(4,4-azo-n-pentyl)-21-deoxyajmalinium, and drugs such as procainamide, desipramine, amantadine, bile acid-cisplatin derivatives such as cis-diammine-chloro-cholylglycinate-platinum(ll)] and cis-diammine-bisursodeoxycholate-platinum(II), azidothymine (AZT) and 2' deoxytubercidin (Arndt, Am. J. Physiol. Renal. Physiol. 281 (2001), F454-468; Dresser, J. Pharm. Sci. 90 (2001), 397-421; Gorboulev, DNA Cell Biol. 16 (1997), 871-881; Kimura, J. Pharmacol. Exp. Ther. 301 (2002), 293-298; van Montfoort, J. Pharmacol. Exp. Ther. 298 (2001), 110-115; Briz, Mol. Pharmacol. 61 (2002), 853-860; http://bigfoot.med.unc.edu/watkinsLab/intesinfo.htm). OCT1 is inhibited by non-transported cations, anions, uncharged compounds and drugs such as prazosin, progesterone, beta oestradiol, phenoxybenzamine, cyanine863 and HIV protease inhibitors indinavir, nefinavir, ritonavir, saquinavir (Arndt, Am. J. Physiol. Renal. Physiol. 281 (2001), F454-468; Zhang, Drug Metab. Dispos. 28 (2000), 329-334; Hayer-Zillgen, Br. J. Pharmacol. 136 (2002), 829-836).

OCT1 plays a major role in hepatic excretion of cations (Briz, Mol. Pharmacol. 61 (2002), 853-860; Dresser, J. Pharm. Sci. 90 (2001), 397-421; Gorboulev, DNA Cell Biol. 16 (1997), 871-881; van Montfoort, J. Pharmacol. Exp. Ther. 298 (2001), 110-115), Koepsell, J. Membr. Biol. 167 (1999), 103-117), participates in the removal of neurotransmitters from the interstitial space (Chen, J. Neurosci. 21 (2001), 6348-6361), mediates cellular release of acetylcholine (Wessler, Br. J. Pharmacol. 134 (2001), 951-956) and participates in the excretion of prostaglandins (Kimura, J. Pharmacol. Exp. Ther. 301 (2002), 293-298).

Many drugs or other treatments are known to have highly variable safety and efficacy in different individuals. A consequence of such variability is that a given drug or other treatment may be effective in one individual, and ineffective or not well-tolerated in another individual. Thus, administration of such a drug to an individual in whom the drug would be ineffective would result in wasted cost and time during which the patient's condition may significantly worsen. Also, administration of a drug to an individual in whom the drug would not be tolerated could result in a direct worsening of the patient's condition and could even result in the patient's death.

The pathway of a certain drug in the body includes the absorption, distribution, metabolism and excretion. For some drugs, over 90% of the measurable interindividual variation in selected phamacokinetic parameters has been shown to be heritable. However, the genetic basis that contributes to the interindividual variation in phamacokinetic parameters is largely identified.

In addition to interindividual variability in pharmakokinetic parameters, another major problem in drug therapy is the occurrence of hepatic side effects as a consequence of exposure to drugs. Hepatotoxicity has been described for a variety of commonly used drugs including nonsteroidal anti-inflammatory drugs, antihypertensives, antidiabetic agents (e.g. gliclazide, troglitazone), anticonvulsants (e.g. valproic acid), lipid-lowering agents such as ,,statins", psychotropic drugs, and antimicrobial agents (Chitturi, Semin. Liver Dis. 22 (2002), 169-83; Brown, Semin Liver Dis 22 (2002), 157-67). Hepatotoxic adverse drug reactions have contributed to the decline of many promising therapies, even among mainstream medication classes (Chitturi, Semin. Liver Dis. 22 (2002)).

Such drugs induced hepatic side effects have frequently phenotypes that resemble or are identical to liver diseases, such as intrahepatic cholestases.

Transport proteins also play a role in drug-induced liver disease and in primary biliary cirrhosis (Jansen, Ned Tijdschr Geneeskd 144 (2000), 2384-91). One important mechanism is the interference with the bile salt export of drugs and their metabolites (i.e. estrogen, cyclosporin A, rifampicin, glibenclamide, rifamycin) that lead to an intracellular accumulation of toxic bile salts with subsequent toxic liver cell necrosis followed by cirrhosis (Stieger, Gastroenterology 118 (2000), 422-30). Hepatic liver damage and cholestasis resulting from drugs is an increasingly recognized cause of liver disease. It produces a broad clinical-pathologic spectrum of injury that includes simple jaundice, cholestatic hepatitis, and bile duct injury that can mimic extrahepatic biliary obstruction, primary biliary cirrhosis, and sclerosing cholangitis with the risk of fatal outcome (Lewis, Clin Liver Dis. 3 (1999), 433-64).

Liver toxicity, such as intrahepatic cholestasis as a side-effect of drug therapy and the clinical manifestation of this condition, jaundice, has been estimated to account for hospitalization in 2 to 5% of the cases for the general population and approaches as much as 20% in the elderly. With the aging of the population and the common occurrence of poly-drug therapy in geriatric patients, it is to be expected that jaundice due to drug-induced intrahepatic cholestasis will become even more prevalent (Feuer, Drug Metabol. Drug Interact. 10 (1992), 1-161). Furthermore, the incidence of drug-induced liver disease appears to be increasing, reflecting the increasing number of new agents that have been introduced into clinical use over the past several decades (Lewis, Med. Clin. North Am. 84 (2000), 1275-311). However, no diagnostic tools are currently available to predict the individual susceptibility to or drug induced liver damage and cholestatic disorders such as drug-induced cholestasis (DIC) prior to onset of the disease condition.

Another increasing problem in drug therapy are drug-drug interactions. As an example, for antiretroviral therapy, especially therapy which is aimed at eradicating the HIV1 virus in the treatment of AIDS, consists of the combined applications of diverse drugs including HIV protease inhibitors such as indinavir, nefinavir, ritonavir or saquinavir. This combination therapy usually involves the simultaneous application of drugs that target viral replication and propagation by inhibition of reverse transcriptase, polymerase, and protease of the virus. Protease inhibitors potently inhibit the transport of cationic drugs, which are substrates for transporters such as OCT1 and lead to potential drug-drug interactions (Zhang, Drug Metab. Dispos. 28 (2000), 329-334). However, so far, the occurrence and degree of said drug-drug interactions caused by interference with the transport of organic cations are not predictable for individual patients.

Human OCT1 is a transporter, that transports a variety of compounds, including drugs. However, nothing is known on the presence of genetic polymorphisms in the OCT1 gene and the impact of such variability on the transport of pharmacological active compounds and their metabolites with its implication for drug safety, tolerability and efficacy.

Thus, means and methods for diagnosing and predicting therapeutic efficacy, or safety of a treatment involving OCT substrates or for diagnosing and treating a variety of diseases and disorders based on dysfunctions or dysregulations of OCT1 were not available yet but are nevertheless highly desirable. Thus, the technical problem underlying the present invention is to comply with the above specified needs.

The solution to this technical problem is achieved by providing the embodiments characterized in the claims.

### Summary of the invention

The present invention relates to a polynucleotide comprising a polynucleotide selected from the group consisting of:
(a) a polynucleotide having the nucleic acid sequence of SEQ ID NO: 1, 2, 3, 4, 5, 6, 7, 8, 9, 10, 11, 12, 13, 14, 15, 16 or 17;
(b) a polynucleotide encoding a polypeptide having the amino acid sequence of SEQ ID NO: 28, 29, 30, 31, 32 or 33;
(c) a polynucleotide having a nucleic acid sequence with at least 70%, preferably at least 75%, at least 80%, at least 85%, at least 90% or at least 95% sequence identity to an OCT1 gene, wherein said polynucleotide is having a nucleotide exchange or a nucleotide deletion of at least one nucleotide at a position 107155, 107265, 107278, 109130, 109211, 119220, 123551, 126806, 126846, 126863 to 126865, 126922, 126915, 130672, 141819, 142951, 141961 or 142993 of the OCT1 gene (GenBank Accession No: Gl:9581607);
(d) a polynucleotide capable of hybridizing to an OCT1 gene, wherein said polynucleotide is having a substitution of at least one nucleotide at a position corresponding to position 107155, 107265, 107278, 109130, 109211, 119220, 123551, 126806, 126846, 126922, 126915, 130672, 141819, 142951, 141961 or 142993 of the OCT1 gene (GenBank Accession No: Gl:9581607 or a deletion of three nucleotides at a position corresponding to position 126863 to 126865 of the OCT1 gene (GenBank Accession No: Gl:9581607);
(e) a polynucleotide capable of hybridizing to an OCT1 gene, wherein said polynucleotide is having an A at a position corresponding to position 107155, 107265, 126806, 141819, 142951 or 142993 of the OCT1 gene (GenBank Accession No: Gl: 9581607), a C at a position corresponding to position 107278, 109211 or 126846 of the OCT1 gene (GenBank Accession No: Gl: 9581607), a G at a position corresponding to position 126922 or 130672 of the OCT1 gene (GenBank Accession No: Gl: 9581607), a T at a position corresponding to position 109130, 119220, 123551, 126915 or 141961 of the OCT1 gene (GenBank Accession No: Gl: 9581607) or an ATG deletion at a position corresponding to position 126863 to 126865 of the OCT1 gene (GenBank Accession No: Gl:9581607);
(f) a polynucleotide encoding an OCT1 polypeptide or fragment thereof, wherein said polypeptide comprises an amino acid substitution at position 61, 88, 401, 414 or 465 of the OCT1 polypeptide (GenBank Accession No:Gl:2511670); and
(g) a polynucleotide encoding an OCT1 polypeptide or fragment thereof, wherein said polypeptide comprises an amino acid substitution of R to C at position 61, an amino acid substitution of C to R at position 88, an amino acid substitution of G to S at position 401, an amino acid substitution of G to A at position 414, an amino acid deletion of M at position 420 or an amino acid substitution of G to R at position 465 of the OCT1 polypeptide (GenBank Accession No: Gl:2511670).

In the context of the present invention the term "polynucleotides" or the term "polypeptides" refers to different variants of a polynucleotide or polypeptide. Said variants comprise a reference or wild type sequence of the polynucleotides or polypeptides of the invention as well as variants which differ therefrom in structure or composition. Reference or wild type sequences for the polynucleotides are GenBank Accession No: Gl:9581607 and Gl:2511669. Reference or wild type sequence for the polypeptides of the invention is GenBank Accession No: Gl:2511670. The differences in structure or composition usually occur by way of nucleotide or amino acid substitution(s) and/or deletion(s). Preferred deletions in accordance with the invention are an ATG deletion at a position corresponding to position 126863 to 126865 of the OCT1 gene (GenBank Accession No: Gl:9581607) and a TGGTAAGT deletion at a position corresponding to position 126880 to 126887 of the OCT1 gene (GenBank Accession No: Gl:9581607).

Preferably, said nucleotide substitution(s) or deletion(s) comprised by the present invention result(s) in one or more changes of the corresponding amino acid(s) of the polypeptides of the invention.

The variant polynucleotides and polypeptides also comprise fragments of said polynucleotides or polypeptides of the invention. The term "polynucleotides" as used herein preferably encompasses the nucleic acid sequences specifically referred to by SEQ ID NOs and in the tables below as well as polynucleotides comprising the reverse complementary nucleic acid sequence thereto. The polynucleotides and polypeptides as well as the aforementioned fragments thereof of the present invention are characterized as being associated with an OCT1 dysfunction or dysregulation comprising, e.g., insufficient and/or altered drug uptake. Said dysfunctions or dysregulations referred to in the present invention cause side effects, reduced activity of drug therapy, or non-response to drug therapy as the result of altered serum and/or intracellular concentrations of compounds that are substrates for OCT1. At least in a subset of subjects said dysfunctions referred to in the present invention may cause a disease or disorder or a prevalence for said disease or disorder. Preferably, as will be discussed below in detail, said disorder results from aberrant serum and/or intracellular concentrations of compounds that are substrates for the transporter OCT1.

The polynucleotides of the invention include polynucleotides that have at least 70%, preferably at least 75%, at least 80%, at least 85%, at least 90% or at least 95% sequence identity to an OCT1 gene, wherein said polynucleotide is having a nucleotide exchange or a nucleotide deletion of at least one nucleotide at a position 107155, 107265, 107278, 109130, 109211, 119220, 123551, 126806, 126846, 126863 to 126865, 126922, 126915, 130672, 141819, 142951, 141961 or 142993 of the OCT1 gene (GenBank Accession No: Gl:9581607).

The term "hybridizing" as used herein refers to polynucleotides which are capable of hybridizing to the polynucleotides of the invention or parts thereof which are associated with an OCT1 dysfunction or dysregulation. Thus, said hybridizing polynucleotides are also associated with said dysfunctions and dysregulations. Preferably, said polynucleotides capable of hybridizing to the polynucleotides of the invention or parts thereof which are associated with OCT1 dysfunctions or dysregulations are at least 70%, at least 80%, at least 95% or at least 100% identical to the polynucleotides of the invention or parts thereof which are associated with OCT1 dysfunctions or dysregulations. Therefore, said polynucleotides may be useful as probes in Northern or Southern Blot analysis of RNA or DNA preparations, respectively, or can be used as oligonucleotide primers in PCR analysis dependent on their respective size. Also comprised by the invention are hybridizing polynucleotides which are useful for analysing DNA-Protein interactions via, e.g., electrophoretic mobility shift analysis (EMSA). Preferably, said hybridizing polynucleotides comprise at least 10, more preferably at least 15 nucleotides in length while a hybridizing polynucleotide of the present invention to be used as a probe preferably comprises at least 100, more preferably at least 200, or most preferably at least 500 nucleotides in length.

It is well known in the art how to perform hybridization experiments with nucleic acid molecules, i.e. the person skilled in the art knows what hybridization conditions s/he has to use in accordance with the present invention. Such hybridization conditions are referred to in standard text books such as Molecular Cloning A Laboratory Manual, Cold Spring Harbor Laboratory (1989) N.Y. Preferred in accordance with the present inventions are polynucleotides which are capable of hybridizing to the polynucleotides of the invention or parts thereof which are associated with an OCT1 dysfunction or dysregulation under stringent hybridization conditions, i.e. which do not cross hybridize to unrelated polynucleotides such as polynucleotides encoding a polypeptide different from the OCT1 polypeptides of the invention.

Nucleic acid hybridization will be affected by such conditions as salt concentration, temperature, or organic solvents, in addition to the base composition, length of the complementary strands and the number of nucleotide base mismatches between the hybridizing nucleic acids, as will be readily appreciated by those skilled in the art. Stringent temperature conditions will generally include temperatures in excess of 30°C, typically 37°C, and preferably in excess of 45°C. Stringent salt conditions will ordinarily be less than 1000 mM, typically less than 500 mM and preferably less than 200 mM. However, the combination of parameters is much more important than the measure of any single parameter; see, e.g., Wetmur and Davidson, 1968. Probe sequences may also hybridize specifically to duplex DNA under certain conditions to form triplex or higher order DNA complexes. The preparation of such probes and suitable hybridization conditions are well known in the art. Polynucleotides which are capable of hybridizing to the polynucleotides of the invention are preferably at least 70%, preferably at least 75%, at least 80%, at least 85%, at least 90% or at least 95% identical to the nucleic acid sequences of the OCT1 gene referred to herein.

The term "percent sequence identity" or "identical" in the context of nucleic acid sequences refers to the residues in the two sequences which are the same when aligned for maximum correspondence. The length of sequence identity comparison may be over a stretch of at least nine nucleotides, usually at least 20 nucleotides, more usually at least 24 nucleotides, typically at least 28 nucleotides, more typically at least 32 nucleotides, and preferably at least 36 nucleotides or more nucleotides. There are a number of different algorithms known in the art which can be used to measure nucleotide sequence identity. For instance, polynucleotide sequences can be compared using Fasta, a program in GCG Version 6.6. Fasta provides alignments and percent sequence identity of the regions of the best overlap between the query and the search sequence (Pearson, 1980, herein incorporated by reference). For instance, percent sequence identity between nucleic acid sequences can be determined using Fasta with its default parameters (a word size of 6 and the NOPAMfactor for the scoring matrix) as provided in GCG Version 6.1, herein incorporated by reference.

The term "corresponding" as used herein means that a position is not only determined by the number of the preceding nucleotides and amino acids, respectively. The position of a given nucleotide or amino acid in accordance with the present invention which may be deleted or substituted vary due to deletions or additional nucleotides or amino acids elsewhere in the gene or the polypeptide. Thus, under a "corresponding position" in accordance with the present invention it is to be understood that nucleotides or amino acids may differ in the indicated number but may still have similar neighboring nucleotides or amino acids. Said nucleotides or amino acids which may be exchanged or deleted nucleotides or amino acids are also comprised by the term "corresponding position". Said nucleotides or amino acids may for instance together with their neighbors form sequences which may be involved in the regulation of gene expression, stability of the corresponding RNA or RNA editing, as well as encode functional domains or motifs of the protein of the invention.

By, e.g., "position 126863 to 126865" it is meant that said polynucleotide comprises one or more deleted nucleotides which are deleted between positions 126863 and position 126865 of the corresponding wild type version of said polynucleotide, e.g. a deletion of three nucleotides. The same applies mutatis mutandis to all other position numbers referred to in the above embodiment which are drafted in the same format.

In accordance with the present invention, the mode and population distribution of genetic variations in the OCT1 gene has been analyzed by sequence analysis of relevant regions of the human said gene from many different individuals. It is a well known fact that genomic DNA of individuals, which harbor the individual genetic makeup of all genes, including the OCT1 gene, can easily be purified from individual blood samples. These individual DNA samples are then used for the analysis of the sequence composition of the alleles of the OCT1 gene that are present in the individual which provided the blood sample. The sequence analysis was carried out by PCR amplification of relevant regions of said genes, subsequent purification of the PCR products, followed by automated DNA sequencing with established methods (e.g. ABl dyeterminator cycle sequencing).

One important parameter that had to be considered in the attempt to determine the individual genotypes and identify novel variants of the OCT1 gene by direct DNA-sequencing of PCR-products from human blood genomic DNA is the fact that each human harbors (usually, with very few abnormal exceptions) two gene copies of each autosomal gene (diploidy). Because of that, great care had to be taken in the evaluation of the sequences to be able to identify unambiguously not only homozygous sequence variations but also heterozygous variations. The details of the different steps in the identification and characterization of novel polymorphisms in the OCT1 gene (homozygous and heterozygous) are described in the Examples below.

Over the past 20 years, genetic heterogeneity has been increasingly recognized as a significant source of variation in drug response. Many scientific communications (Meyer, Ann. Rev. Pharmacol. Toxicol. 37 (1997), 269-296 and West, J. Clin. Pharmacol. 37 (1997), 635-648) have clearly shown that some drugs work better or may even be highly toxic in some patients than in others and that these variations in patient's responses to drugs can be related to molecular basis. This "pharmacogenomic" concept spots correlations between responses to drugs and genetic profiles of patient's (Marshall, Nature Biotechnology, 15 (1997), 954-957; Marshall, Nature Biotechnology, 15 (1997), 1249-1252). In this context of population variability with regard to drug therapy, pharmacogenomics has been proposed as a tool useful in the identification and selection of patients which can respond to a particular drug without side effects. This identification/selection can be based upon molecular diagnosis of genetic polymorphisms by genotyping DNA from leukocytes in the blood of patient, for example, and characterization of disease (Bertz, Clin. Pharmacokinet. 32 (1997), 210-256; Engel, J. Chromatogra. B. Biomed. Appl. 678 (1996), 93-103). For the founders of health care, such as health maintenance organizations in the US and government public health services in many European countries, this pharmacogenomics approach can represent a way of both improving health care and reducing overheads because there is a large cost to unnecessary drugs, ineffective drugs and drugs with side effects.

The mutations in the variant genes of the invention sometime result in amino acid deletion(s), insertion(s) and in particular in substitution(s) either alone or in combination. It is of course also possible to genetically engineer such mutations in wild type genes or other mutant forms. Methods for introducing such modifications in the DNA sequence of said genes are well known to the person skilled in the art; see, e.g., Sambrook, Molecular Cloning A Laboratory Manual, Cold Spring Harbor Laboratory (1989) N.Y.

For the investigation of the nature of the alterations in the amino acid sequence of the polypeptides of the invention computer programs may be used such as BRASMOL that are obtainable from the Internet. Furthermore, folding simulations and computer redesign of structural motifs can be performed using other appropriate computer programs (Olszewski, Proteins 25 (1996), 286-299; Hoffman, Comput. Appl. Biosci. 11 (1995), 675-679). Computers can be used for the conformational and energetic analysis of detailed protein models (Monge, J. Mol. Biol. 247 (1995), 995-1012; Renouf, Adv. Exp. Med. Biol. 376 (1995), 37-45). These analysis can be used for the identification of the influence of a particular mutation on binding and/or transport of drugs by OCT1, or its influence on the folding or stability of the protein.

Usually, said amino acid deletion or substitutions in the amino acid sequence of the protein encoded by the polynucleotide of the invention is due to one or more nucleotide substitution or deletion, or any combinations thereof. Preferably said nucleotide substitution or deletion may result in an amino acid substitution of R to C at position corresponding to position 61 of the OCT1 polypeptide (GenBank Accession No: Gl:2511670), an amino acid substitution of C to R at position corresponding to position 88 of the OCT1 polypeptide (GenBank Accession No: Gl:2511670) and an amino acid substitution of G to S at position corresponding to position 401 of the OCT1 polypeptide (GenBank Accession No: Gl:2511670). The polypeptides encoded by the polynucleotides of the invention have altered biological or immunological properties due to the mutations referred to in accordance with the present invention. Examples for said altered properties are altered substrate specificity or an altered transport activity characterized by, e.g., insufficiencies in drug transport or a complete loss of the capability of transporting some or all drugs that are substrate for the wild-type OCT1 protein.

The mutations in the OCT1 gene detected in accordance with the present invention are listed in Tables 1 to 4. The methods of the mutation analysis followed standard protocols and are described in detail in the Examples and references cited in the present invention. In general such methods are to be used in accordance with the present invention for evaluating the phenotypic spectrum as well as the overlapping clinical characteristics of diseases or conditions related to dysfunctions or dysregulations and diseases related to altered drug transport. Advantageously, the characterization of said mutants may form the basis of the development of diagnostic assays for the improved therapy with drugs that are substrates of OCT1, or with drugs that act on or interfere with biological pathways associated with substrates of OCT1 such as indicated above (e.g. serotonin, acetylcholine etc.). Thanks to the present invention polymorphisms have been found which result in an altered drug uptake and altered substrate specificity of the OCT1 transporter protein. This may have important biomedical implications. As a consequence of altered pharmacokinetics an enhanced duration and intensity of a drug with implication for drug efficacy, safety, and tolerability can be anticipated in carriers of these mutations.

Further, according to the present invention, polymorphisms in the OCT1 gene have been identified that are associated with hepatic side effects and cholestasis. Thus, the genotyping of the OCT1 gene will be useful for the diagnosis of subjects with an inscreased risk for suffering of diseases such as hepatotoxicity and cholestasis. Thanks to the present invention, subjects can be identified that should be monitored to prevent a serious liver disease or may be preselected for altered drug therapy. The genotype will rarely be absolutely predictive, i.e., where a population with a certain genotype displays a high incidence of a particular phenotype, not every individual with that genotype will display the phenotype. However, it will be apparent to the person skilled in the art that genotyping a subject as described herein will be an aid in predicting the outcome a subject will have to treatment with an OCT1 substrate.

According to the present invention, the mutants of the OCT1 gene may contribute to the individual variability of drug interactions in the course of anti-retroviral therapy including HIV therapy. Different components of anti-retroviral therapy are either inhibitors (e.g. saquinavir, nelfinavir, indinavir, ritonavir) or substrates of the OCT1 transporter protein such as AZT. Thus the genotyping of the OCT1 mutants will be useful for predicting the cellular uptake and distribution of OCT1 substrates, e.g. the OCT1 activity and subsequent drug response.

More preferably, the diagnosis of said OCT1 polymorphisms will be useful for association of the OCT1 variants of the present invention with the individual response and/or side effects during anti-retroviral therapy, i.e. will allow to predict the occurances and degrees of drug-drug interactions depending on the genetic constitution of the OCT1 gene. This OCT1 diagnosis, in turn, opens the possibility to compensate for the predicted drug-drug interactions.

Said methods for the analysis of mutations encompass, for example, DNA sequencing, hybridisation techniques, PCR based assays, fluorescent dye and quenching agent-based PCR assay (Taqman PCR detection system), RFLP-based techniques, single strand conformational polymorphism (SSCP), denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, DHPLC-based techniques, oligonucleotide extension assays (OLA), extension based assays (ARMS, (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), pyrosequencing, protein truncation assay (PTT), immunoassays, haplotype analysis, and solid phase hydridization (dot blot, reverse dot blot, chips). Said techniques are very well known in the art and described, e.g., in Siitari, Nucleic acid diagnostics market, Technology Review 125/2002, ISDN 1239-758X, Caplin, Biochemica 1 (1999), 5-8; Neville, BioTechniques 32 (2002), 34-43; Shi 47 (2001), 164-72, Underhill, Genome Res 7 (1997), 996-1005; Oefner, J Chromatogr B Biomed Sci Appl 739 (2000), 345-55, the patent application US 20010049586. Moreover, kits for carrying out these techniques may be commercially available from, e.g., Applied biosystems. On the basis of thorough clinical characterization of many patients the phenotypes can then be correlated to these mutations.

Also comprised by the polynucleotides referred to in the present invention are polynucleotides which comprise at least two of the polynucleotides specified hereinabove, i.e. polynucleotides having a nucleotide sequence which contains at least two of the mutations comprised by the above polynucleotides or listed in Tables 1 to 4 and Table 6 below. Said polynucleotides of the present invention are further referred to as alleles and haplotypes. Those mutations or variants comprised by the above polynucleotides may be either a marker polymorphism or a functional polymorphism. These variants can be used in many aspects of genetic analysis and diagnosis including genetic disease and population studies. Two types of genetic analyses are typically performed: linkage and association studies.

Defined genetic variations of genes can directly be associated with corresponding phenotypes in some cases. In many other cases, however, it is known that the determination of haplotypes is more predictive of a phenotype than the determination of single polymorphisms (Judson, Pharmacogenomics 1 (2000), 15-26 Judson, Pharmacogenomics 2 (2001), 7-10; Bader, Pharmacogenomics. 2 (2001), 11-24). It is well known to experts in the art how to perform haplotying. Beside molecular haplotyping computer programs can be used for haplotype analysis; see, e.g., ftp://linkage.rockefeller.edu/software/eh; www.bioinf.mdc-berlin.de/projects/hap.

Preferred haplotypes are the Met408Val polymorphism (SEQ ID NO:24, 35) that is linked to a deletion of TGGTAAGT at position 17939 of the OCT1 gene (SEQ ID NO: 21), the SNP 33012G>T in intron 9 (SEQ ID NO: 16) is linked to the 34044G>A mutant in exon 10 (SEQ ID NO: 17), the -1795G>A substitution in the promoter of the OCT1 gene (SEQ ID NO: 1) is linked to the 156T>C variation in exon 1 (SEQ ID NO: 22), and the 10270C>T variant in intron 2 (SEQ ID NO: 6) to 14602C>T substitution in intron 5 (SEQ ID NO: 7). Most preferred haplotypes are the Met408Va1 polymorphism (SEQ ID NO:24, 35) that is linked to a deletion of TGGTAAGT at position 17939 of the OCT1 gene (SEQ ID NO:21), Obviously, other so far undiscovered- SNPs can also be present in the larger region of these defined haplotypes. This allows the study of synergistic effects of said mutations in the OCT1 gene and/or a polypeptide encoded by said polynucleotide on the pharmacological profile of drugs in patients who bear such mutant forms of the gene or similar mutant forms that can be mimicked by the above described proteins. It is expected that the analysis of said synergistic effects provides deeper insights into the onset of OCT1 dysfunctions or dysregulations or diseases related to altered drug transport as described supra. From said deeper insight the development of diagnostic and pharmaceutical compositions related to OCT1 dysfunctions or dysregulations or diseases related to altered transport will greatly benefit.

The term "allele" in the context of the present invention can be defined by the particular nucleotide(s) present in a nucleic acid sequence from a subject or a patient at a particular site(s). Often a genotype is the nucleotide(s) present at a single polymorphic site known to vary in the human population.

In the context of the present invention, the term "haplotype" means a cis arrangement of two or more polymorphic nucleotides, i.e., mutants or variants, on a particular chromosome, e.g., in a particular gene. The haplotypes contains information about the phases of the polymorphic nucleotides, that means, which set of mutants or variants were inherited from the father and which from the mother.

As is evident to the person skilled in the art, the genetic knowledge deduced from the present invention can now be used to exactly and reliably characterize the genotype of a patient. Advantageously, OCT1 dysfunction or dysregulation resulting from aberrant serum and/or intracellular concentrations of compounds that are substrates of the transporter OCT1 and/or diseases or a prevalence for a disease which are associated with OCT1 dysfunction or dysregulation referred to herein can be predicted and preventive or therapeutical measures can be applied accordingly. Moreover in accordance with the foregoing, in cases where a given drug takes an unusual effect, a suitable individual therapy can be designed based on the knowledge of the individual genetic makeup of a subject with respect to the polynucleotides of the invention and improved therapeutics can be developed as will be further discussed below.

In general, the OCT1 "status", defined by the expression level and activity of the OCT1 protein, can be not only altered in many disease or disorders including disorders resulting from aberrant serum and/or intracellular concentrations of compounds that are substrates of the transporter OCT1, (see above), but can also be variable in normal tissue, due to genetic variations/polymorphisms. The identification of polymorphisms associated with altered OCT1 expression and/or activity is important for the prediction of e.g. drug uptake and transport, and subsequently for the prediction of therapy outcome, including side effects of medications. Therefore, analysis of OCT1 variations indicative of OCT1 function, is a valuable tool for therapy with drugs, which are substrates of OCT1 and has, thanks to the present invention, now become possible.

Finally, the polynucleotides and polypeptides referred to in accordance with the present invention are also useful as forensic markers, which improve the identification of subjects which have been murdered or killed by, for example a crime of violence or any other violence and can not be identified by the well known conventional forensic methods. The application of forensic methods based on the detection of the polymorphisms comprised by the polynucleotides of this invention in the genome of a subject are particularly well suited in cases where a (dead) body is disfigured in a severe manner such as identification by other body characteristics such as the features of the face is not possible. This is the case, for example, for corpses found in water which are usually entirely disfigured. Advantageously, methods which are based on the provision of the polynucleotides of the invention merely require a minimal amount of tissue or cells in order to be carried out. Said tissues or cells may be blood droplets, hair roots, epidermal scales, salivia droplets, sperms etc. Since only such a minimal amount of tissue or cells is required for the identification of a subject, the polymorphism comprised by the polynucleotides of this invention can also be used as forensic markers in order to proof someone guilty for a crime, such as a violation or a ravishment. Moreover, the polymorphisms comprised by the polynucleotides of this invention can be used to proof paternity. In accordance with the forensic methods referred herein the presence or absence of the polynucleotides of the invention is determined and compared with a reference sample which is unambiguously derived from the subject to be identified. The forensic methods which require detection of the presence or absence of the polynucleotides of this invention in a sample of a subject the polymorphisms comprised by the polynucleotides of this invention can be for example PCR-based techniques which are particularly well suited in cases where only minimal amount of tissue or cells is available as forensic samples. On the other hand, where enough tissue or cells is available, hybridization based techniques may be performed in order to detect the presence or absence of a polynucleotide of this invention. These techniques are well known by the person skilled in the art and can be adopted to the individual purposes referred to herein without further ado. In conclusion, thanks to the present invention forensic means which allow improved and reliable predictions as regards the aforementioned aspects are now available.

In line with the foregoing, preferably, the polynucleotide of the present invention is associated with side effects, or reduced activity of drug therapy, or non-activity of drug therapy resulting from aberrant serum and/or intracellular concentrations of compounds that are substrates of the transporter OCT1.

In a further embodiment the present invention relates to a polynucleotide which is DNA or RNA.

The polynucleotide of the invention may be, e.g., DNA, cDNA, genomic DNA, RNA or synthetically produced DNA or RNA or a recombinantly produced chimeric nucleic acid molecule comprising any of those polynucleotides either alone or in combination. Preferably said polynucleotide is part of a vector, particularly plasmids, cosmids, viruses and bacteriophages used conventionally in genetic engineering that comprise a polynucleotide of the invention. Such vectors may comprise further genes such as marker genes which allow for the selection of said vector in a suitable host cell and under suitable conditions.

The invention furthermore relates to a gene comprising the polynucleotide of the invention.

It is well known in the art that genes comprise structural elements which encode an amino acid sequence as well as regulatory elements which are involved in the regulation of the expression of said genes. Structural elements are represented by exons which may either encode an amino acid sequence or which may encode for RNA which is not encoding an amino acid sequence but is nevertheless involved in RNA function, e.g. by regulating the stability of the RNA or the nuclear export of the RNA.

Regulatory elements of a gene may comprise promoter elements or enhancer elements both of which could be involved in transcriptional control of gene expression. It is very well known in the art that a promoter is to be found upstream of the structural elements of a gene. Regulatory elements such as enhancer elements, however, can be found distributed over the entire locus of a gene. Said elements could be reside, e.g., in introns, regions of genomic DNA which separate the exons of a gene. Promoter or enhancer elements correspond to polynucleotide fragments which are capable of attracting or binding polypeptides involved in the regulation of the gene comprising said promoter or enhancer elements. For example, polypeptides involved in regulation of said gene comprise the so called transcription factors.

Said introns may comprise further regulatory elements which are required for proper gene expression. lntrons are usually transcribed together with the exons of a gene resulting in a nascent RNA transcript which contains both, exon and intron sequences. The intron encoded RNA sequences are usually removed by a process known as RNA splicing. However, said process also requires regulatory sequences present on a RNA transcript said regulatory sequences may be encoded by the introns.

In addition, besides their function in transcriptional control and control of proper RNA processing and/or stability, regulatory elements of a gene could be also involved in the control of genetic stability of a gene locus. Said elements control, e.g., recombination events or serve to maintain a certain structure of the DNA or the arrangement of DNA in a chromosome.

Therefore, single nucleotide polymorphisms can occur in exons of a gene which encode an amino acid sequence as discussed supra as well as in regulatory regions which are involved in the above discussed process. The analysis of the nucleotide sequence of a gene locus in its entirety including, e.g., introns is in light of the above desirable. The polymorphisms comprised by the polynucleotides of the present invention can influence the expression level of OCT1 protein via mechanisms involving enhanced or reduced transcription of the OCT1 gene, stabilization of the gene's RNA transcripts and alteration of the processing of the primary RNA transcripts.

Therefore, in a furthermore preferred embodiment of the gene of the invention a nucleotide deletion and/or substitution results in altered expression of the variant gene compared to the corresponding wild type gene.

In another embodiment the present invention relates to a vector comprising the polynucleotide of the invention or the gene of the invention.

Said vector may be, for example, a phage, plasmid, viral or retroviral vector. Retroviral vectors may be replication competent or replication defective. In the latter case, viral propagation generally will occur only in complementing host/cells.

The polynucleotides or genes of the invention may be joined to a vector containing selectable markers for propagation in a host. Generally, a plasmid vector is introduced in a precipitate such as a calcium phosphate precipitate, using the DEAE-Method, in a condensed form using chemicals such as effectene (Qiagen, Hilden, Germany), or in a complex with a charged lipid or in carbon-based clusters. Alternatively, the vector is introduced via microinjection. Should the vector be a virus, it may be packaged in vitro using an appropriate packaging cell line prior to application to host cells.

In a more preferred embodiment of the vector of the invention the polynucleotide is operatively linked to expression control sequences allowing expression in prokaryotic or eukaryotic cells or isolated fractions thereof.

Expression of said polynucleotide comprises transcription of the polynucleotide, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the lac, *trp* or tac promoter in *E. coli,* and examples for regulatory elements permitting expression in eukaryotic host cells are the *AOX1* or *GAL1* promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (Invitrogen), pSPORT1 (GIBCO BRL), pFastBac (Invitrogen), pYES (Invitrogen), pOG1 (van Montfoort, JPET 298 (2001), 110-115). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Alternatively, the polynucleotides and vectors of the invention can be reconstituted into liposomes for delivery to target cells.

The term "isolated fractions thereof" refers to fractions of eukaryotic or prokaryotic cells or tissues which are capable of transcribing or transcribing and translating RNA from the vector of the invention. Said fractions comprise proteins which are required for transcription of RNA or transcription of RNA and translation of said RNA into a polypeptide. Said isolated fractions may be, e.g., nuclear and cytoplasmic fractions of eukaryotic cells such as of reticulocytes.

The present invention furthermore relates to a host cell genetically engineered with the polynucleotide of the invention, the gene of the invention or the vector of the invention.

Said host cell may be a prokaryotic or eukaryotic cell; see supra. The polynucleotide or vector of the invention which is present in the host cell may either be integrated into the genome of the host cell or it may be maintained extrachromosomally. In this respect, it is also to be understood that the recombinant DNA molecule of the invention can be used for "gene targeting" and/or "gene replacement", for restoring a mutant gene or for creating a mutant gene via homologous recombination; see for example Mouellic, Proc. Natl. Acad. Sci. USA, 87 (1990), 4712-4716; Joyner, Gene Targeting, A Practical Approach, Oxford University Press.

The host cell can be any prokaryotic or eukaryotic cell, such as a bacterial, insect, fungal, plant, animal, mammalian or, preferably, human cell. Preferred fungal cells are, for example, those of the genus Saccharomyces, in particular those of the species S. cerevisiae. Preferred animal cells are, for example, *Xenopus oocytes.* The term "prokaryotic" is meant to include all bacteria which can be transformed or transfected with a polynucleotide for the expression of a variant polypeptide of the invention. Prokaryotic hosts may include gram negative as well as gram positive bacteria such as, for example, *E. coli,* S. *typhimurium, Serratia marcescens* and *Bacillus subtilis.* A polynucleotide coding for a mutant form of variant polypeptides of the invention can be used to transform or transfect the host using any of the techniques commonly known to those of ordinary skill in the art. Methods for preparing fused, operably linked genes and expressing them in bacteria or animal cells are well-known in the art (Sambrook, supra). The genetic constructs and methods described therein can be utilized for expression of variant polypeptides of the invention in, e.g., prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. The transformed prokaryotic hosts can be grown in fermentors and cultured according to techniques known in the art to achieve optimal cell growth. The proteins of the invention can then be isolated from the grown medium, cellular lysates, or cellular membrane fractions. The isolation and purification of the microbially or otherwise expressed polypeptides of the invention may be by any conventional means such as, for example, preparative chromatographic separations and immunological separations such as those involving the use of monoclonal or polyclonal antibodies.

Thus, in a further embodiment the invention relates to a method for producing a molecular variant OCT1 polypeptide or fragment thereof comprising culturing the above described host cell; and recovering said protein or fragment from the culture.

In another embodiment the present invention relates to a method for producing cells capable of expressing a molecular variant OCT1 polypeptide comprising genetically engineering cells with the polynucleotide of the invention, the gene of the invention or the vector of the invention.

The cells obtainable by the method of the invention can be used, for example, to test drugs according to the methods described in D. L. Spector, R. D. Goldman, L. A. Leinwand, Cells, a Lab manual, CSH Press 1998. Furthermore, the cells can be used to study known drugs and unknown derivatives thereof for their ability to complement the deficiency caused by mutations in the OCT1 gene. For these embodiments the host cells preferably lack a wild type allele, preferably both alleles of the OCT1 gene and/or have at least one mutated from thereof. Ideally, the gene comprising an allele as comprised by the polynucleotides of the invention could be introduced into the wild type locus by homologous replacement. Alternatively, strong overexpression of a mutated allele over the normal allele and comparison with a recombinant cell line overexpressing the normal allele at a similar level may be used as a screening and analysis system. The cells obtainable by the above-described method may also be used for the screening methods referred to herein below.

Furthermore, the invention relates to a polypeptide or fragment thereof encoded by the polynucleotide of the invention, the gene of the invention or obtainable by the method described above or from cells produced by the method described above.

In this context it is also understood that the variant polypeptide of the invention can be further modified by conventional methods known in the art. By providing said variant proteins according to the present invention it is also possible to determine the portions relevant for their biological activity or inhibition of the same. The terms "polypeptide" and "protein" as used herein are exchangeable. Moreover, what is comprised by said terms is standard textbook knowledge.

The present invention furthermore relates to an antibody which binds specifically to the polypeptide of the invention.

Advantageously, the antibody specifically recognizes or binds an epitope containing one or more amino acid substitution(s) as defined above. Antibodies against the variant polypeptides of the invention can be prepared by well known methods using a purified protein according to the invention or a (synthetic) fragment derived therefrom as an antigen. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals. In a preferred embodiment of the invention, said antibody is a monoclonal antibody, a polyclonal antibody, a single chain antibody, human or humanized antibody, primatized, chimerized or fragment thereof that specifically binds said peptide or polypeptide also including bispecific antibody, synthetic antibody, antibody fragment, such as Fab, Fv or scFv fragments etc., or a chemically modified derivative of any of these. Furthermore, antibodies or fragments thereof to the aforementioned polypeptides can be obtained by using methods which are described, e.g., in Harlow and Lane "Antibodies, A Laboratory Manual", CSH Press, Cold Spring Harbor, 1988. These antibodies can be used, for example, for the immunoprecipitation and immunolocalization of the variant polypeptides of the invention as well as for the monitoring of the presence of said variant polypeptides, for example, in recombinant organisms, and for the identification of compounds interacting with the proteins according to the invention. For example, surface plasmon resonance as employed in the BlAcore system can be used to increase the efficiency of phage antibodies which bind to an epitope of the protein of the invention (Schier, Human Antibodies Hybridomas 7 (1996), 97-105; Malmborg, J. Immunol. Methods 183 (1995), 7-13).

In a preferred embodiment the antibody of the present invention specifically recognizes an epitope containing one or more amino acid substitution(s) resulting from a nucleotide exchange as defined supra.

Antibodies which specifically recognize modified amino acids such as phospho-Tyrosine residues are well known in the art. Similarly, in accordance with the present invention antibodies which specifically recognize even a single amino acid exchange in an epitope may be generated by the well known methods described supra.

In light of the foregoing, in a more preferred embodiment the antibody of the present invention is monoclonal or polyclonal.

The invention also relates to a transgenic non-human animal comprising at least one polynucleotide of the invention, the gene of the invention or the vector of the invention as described supra.

The present invention also encompasses a method for the production of a transgenic non-human animal comprising introduction of a polynucleotide or vector of the invention into a germ cell, an embryonic cell, stem cell or an egg or a cell derived therefrom. The non-human animal can be used in accordance with the method of the invention described below and may be a non-transgenic healthy animal, or may have a disease or disorder, preferably a disease caused by at least one mutation in the gene of the invention. Such transgenic animals are well suited for, e.g., pharmacological studies of drugs in connection with variant forms of the above described variant polypeptides since these polypeptides or at least their functional domains are conserved between species in higher eukaryotes, particularly in mammals. Production of transgenic embryos and screening of those can be performed, e.g., as described by A. L. Joyner Ed., Gene Targeting, A Practical Approach (1993), Oxford University Press. The DNA of the embryos can be analyzed using, e.g., Southern blots with an appropriate probe or based on PCR techniques.

A transgenic non-human animal in accordance with the invention may be a transgenic mouse, rat, hamster, dog, monkey, rabbit, pig, frog, nematode such as Caenorhabditis elegans, fruitfly such as Drosophila melanogaster or fish such as torpedo fish or zebrafish comprising a polynucleotide or vector of the invention or obtained by the method described above, preferably wherein said polynucleotide or vector is stably integrated into the genome of said non-human animal, preferably such that the presence of said polynucleotide or vector leads to the expression of the variant polypeptide of the invention. It may comprise one or several copies of the same or different polynucleotides or genes of the invention. This animal has numerous utilities, including as a research model for cardiovascular research and therefore, presents a novel and valuable animal in the development of therapies, treatment, etc. for diseases caused by cardiovascular diseases. Accordingly, in this instance, the mammal is preferably a laboratory animal such as a mouse or rat.

Thus, in a preferred embodiment the transgenic non-human animal of the invention is a mouse, a rat or a zebrafish.

Numerous reports revealed that said animals are particularly well suited as model organisms for the investigation of the drug metabolism and transport and its deficiencies or cancer. Advantageously, transgenic animals can be easily created using said model organisms, due to the availability of various suitable techniques well known in the art.

The invention also relates to a solid support comprising one or a plurality of the polynucleotide, the gene, the vector, the polypeptide, the antibody or the host cell of the invention in immobilized form.

The term "solid support" as used herein refers to a flexible or non-flexible support that is suitable for carrying said immobilized targets. Said solid support may be homogenous or inhomogeneous. For example, said solid support may consist of different materials having the same or different properties with respect to flexibility and immobilization, for instance, or said solid support may consist of one material exhibiting a plurality of properties also comprising flexibility and immobilization properties. Such supports are well known in the art and comprise, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. Said solid support may comprise glass-, polypropylene- or silicon-chips, membranes oligonucleotide-conjugated beads or bead arrays.

The term "immobilized" means that the molecular species of interest is fixed to a solid support, preferably covalently linked thereto. This covalent linkage can be achieved by different means depending on the molecular nature of the molecular species. Moreover, the molecular species may be also fixed on the solid support by electrostatic forces, hydrophobic or hydrophilic interactions, Van-der-Waals forces or photolithography. The above described physico-chemical interactions typically occur in interactions between molecules. For example, biotinylated polypeptides may be fixed on a avidin-coated solid support due to interactions of the above described types. Further, polypeptides such as antibodies, may be fixed on an antibody coated solid support. Moreover, the immobilization is dependent on the chemical properties of the solid support. For example, the nucleic acid molecules can be immobilized on a membrane by standard techniques such as UV-crosslinking, photolithography or heat.

In a preferred embodiment of the invention said solid support is a membrane, a glass- or poylpropylene- or silicon-chip, are oligonucleotide-conjugated beads or a bead array, which is assembled on an optical filter substrate.

Moreover, the present invention relates to an in vitro method for identifying a polymorphism said method comprising the steps of:
(a) isolating a polynucleotide or the gene of the invention from a plurality of subgroups of individuals, wherein one subgroup has no prevalence for an OCT1 associated disease and at least one or more further subgroup(s) do have prevalence for an OCT1 associated disease; and
(b) identifying a polymorphism by comparing the nucleic acid sequence of said polynucleotide or said gene of said one subgroup having no prevalence for an OCT1 associated disease with said at least one or more further subgroup(s) having a prevalence for an OCT1 associated disease.

The term "prevalence" as used herein means that individuals are be susceptible for one or more disease(s) which are associated with OCT1 dysfuntion or dysregulation or could already have one or more of said disease(s). Thereby, one OCT1 associated disease can be used to determine the susceptibility for another OCT1 associated disease, e.g. altered drug transport by OCT1 variants may be indicative for a prevalence for, e.g. disorders resulting from aberrant serum and/or intracellular concentrations of compounds that are substrates of the transporter OCT1. Moreover, symptoms which are indicative for a prevalence for developing said diseases are very well known in the art and have been sufficiently described in standard textbooks of medicine such as Pschyrembel, Stedman and Harrisons's (Principles of internal medicine 15th edition (2001), McGraw Hill, ISBN 0-07-0025113490).

Advantageously, polymorphisms according to the present invention which are associated with OCT1 dysfunction or dysregulation or one or more disease(s) based thereon should be enriched in subgroups of individuals which have a prevalence for said diseases versus subgroups which have no prevalence for said diseases. Thus, the above described method allows the rapid and reliable detection of polymorphism which are indicative for one or more OCT1 associated disease(s) or a susceptibility therefor. Advantageously, due to the phenotypic preselection a large number of individuals having no prevalence might be screened for polymorphisms in general. Thereby, a reference sequences comprising polymorphisms which do not correlate to one or more OCT1 associated disease(s) can be obtained. Based on said reference sequences it is possible to efficiently and reliably determine the relevant polymorphisms.

In a further embodiment the present invention relates to a method for identifying and obtaining a pro-drug or a drug capable of modulating the activity of a molecular variant of an OCT1 polypeptide comprising the steps of:
(a) contacting the polypeptide, the solid support of the invention, a cell expressing a molecular variant gene comprising a polynucleotide of the invention, the gene or the vector of the invention in the presence of components capable of providing a detectable signal in response to drug activity with a compound to be screened for pro-drug or drug activity; and
(b) detecting the presence or absence of a signal or increase or decrease of a signal generated from the pro-drug or the drug activity, wherein the absence, presence, increase or decrease of the signal is indicative for a putative pro-drug or drug.

The term "compound" in a method of the invention includes a single substance or a plurality of substances which may or may not be identical.

Said compound(s) may be chemically synthesized or produced via microbial fermentation but can also be comprised in, for example, samples, e.g., cell extracts from, e.g., plants, animals or microorganisms. Furthermore, said compounds may be known in the art but hitherto not known to be useful as an inhibitor, respectively. The plurality of compounds may be, e.g., added to the culture medium or injected into a cell or non-human animal of the invention.

If a sample containing (a) compound(s) is identified in the method of the invention, then it is either possible to isolate the compound from the original sample identified as containing the compound, in question or one can further subdivide the original sample, for example, if it consists of a plurality of different compounds, so as to reduce the number of different substances per sample and repeat the method with the subdivisions of the original sample. It can then be determined whether said sample or compound displays the desired properties, for example, by the methods described herein or in the literature (Spector et al., Cells manual; see supra). Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art or by using and modifying the methods as described herein. Furthermore, the person skilled in the art will readily recognize which further compounds may be used in order to perform the methods of the invention, for example, enzymes, if necessary, that convert a certain compound into a precursor. Such adaptation of the method of the invention is well within the skill of the person skilled in the art and can be performed without undue experimentation.

Compounds which can be used in accordance with the present invention include peptides, proteins, nucleic acids, antibodies, small organic compounds, ligands, peptidomimetics, PNAs and the like. Said compounds may act as agonists or antagonists of the inveniton. Said compounds can also be functional derivatives or analogues of known drugs. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described. Furthermore, peptide mimetics and/or computer aided design of appropriate drug derivatives and analogues can be used, for example, according to the methods described below. Such analogs comprise molecules may have as the basis structure of known OCT1 substrates and/or inhibitors and/or modulators; see infra.

Appropriate computer programs can be used for the identification of interactive sites of a putative inhibitor and the polypeptides of the invention by computer assistant searches for complementary structural motifs (Fassina, lmmunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known inhibitors, analogs, antagonists or agonists. Appropriate peptidomimetics and other inhibitors can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of said compounds and the polypeptides of the invention can be used for the design of peptidomimetic drugs (Rose, Biochemistry 35 (1996), 12933-12944; Rutenber, Bioorg. Med. Chem. 4 (1996), 1545-1558). It is very well known how to obtain said compounds, e.g. by chemical or biochemical standard techniques. Thus, also comprised by the method of the invention are means of making or producing said compounds. In summary, the present invention provides methods for identifying and obtaining compounds which can be used in specific doses for the treatment of specific forms of OCT1 associated disorders that results from aberrant serum and/or intracellular concentrations of compounds that are substrates of the transporter OCT1.

The above definitions apply mutatis mutandis to all of the methods described in the following.

In a further embodiment the present invention relates to a method for identifying and obtaining an inhibitor of the activity of a molecular variant of an OCT1 polypeptide comprising the steps of:
(a) contacting the protein, the solid support of the invention or a cell expressing a molecular variant gene comprising a polynucleotide or the gene or the vector of the invention in the presence of components capable of providing a detectable signal in response to drug activity with a compound to be screened for inhibiting activity; and
(b) detecting the presence or absence of a signal or increase or decrease of a signal generated from the inhibiting activity, wherein the absence or decrease of the signal is indicative for a putative inhibitor.

In a preferred embodiment of the method of the invention said cell is a cell, obtained by the method of the invention or can be obtained from the transgenic non-human animal as described supra.

In a still further embodiment the present invention relates to a method of identifying and obtaining a pro-drug or drug capable of modulating the activity of a molecular variant of an OCT1 polypeptide comprising the steps of:
(a) contacting the host cell, the cell obtained by the method of the invention, the polypeptide or the solid support of the invention with the first molecule known to be bound by an OCT1 polypeptide to form a first complex of said polypeptide and said first molecule;
(b) contacting said first complex with a compound to be screened; and
(c) measuring whether said compound displaces said first molecule from said first complex.

Advantageously, in said method said measuring step comprises measuring the formation of a second complex of said protein and said inhibitor candidate. Preferably, said measuring step comprises measuring the amount of said first molecule that is not bound to said protein.

In a particularly preferred embodiment of the above-described method of said first molecule is a agonist or antagonist or a substrate and/or a inhibitor and/or a modulator of the polypeptide of the invention, e.g., with a radioactive or fluorescent label.

In a still another embodiment the present invention relates to a method of identifying and obtaining an inhibitor capable of modulating the activity of a molecular variant of an OCT1 polypeptide comprising the steps of:
(a) contacting the host cell or the cell obtained by the method of the invention, the protein or the solid support of the invention with the first molecule known to be bound by the OCT1 polypeptide to form a first complex of said protein and said first molecule;
(b) contacting said first complex with a compound to be screened; and
(c) measuring whether said compound displaces said first molecule from said first complex.

In a preferred embodiment of the method of the invention said measuring step comprises measuring the formation of a second complex of said protein and said compound.

In another preferred embodiment of the method of the invention said measuring step comprises measuring the amount of said first molecule that is not bound to said protein.

In a more preferred embodiment of the method of the invention said first molecule is labeled.

The invention furthermore relates to a method for the production of a pharmaceutical composition comprising the steps of the method as described supra; and the further step of formulating the compound identified and obtained or a derivative thereof in a pharmaceutically acceptable form.

The therapeutically useful compounds identified according to the methods of the invention can be formulated and administered to a patient as discussed above. For uses and therapeutic doses determined to be appropriate by one skilled in the art and for definitions of the term "pharmaceutical composition" see infra.

Furthermore, the present invention encompasses a method for the preparation of a pharmaceutical composition comprising the steps of the above-described methods; and formulating a drug or pro-drug in the form suitable for therapeutic application and preventing or ameliorating the disorder of the subject diagnosed in the method of the invention.

Drugs or pro-drugs after their *in vivo* administration are metabolized in order to be eliminated either by excretion or by metabolism to one or more active or inactive metabolites (Meyer, J. Pharmacokinet. Biopharm. 24 (1996), 449-459). Thus, rather than using the actual compound or inhibitor identified and obtained in accordance with the methods of the present invention a corresponding formulation as a pro-drug can be used which is converted into its active in the patient. Precautionary measures that may be taken for the application of pro-drugs and drugs are described in the literature; see, for review, Ozama, J. Toxicol. Sci. 21 (1996), 323-329).

In a preferred embodiment of the method of the present invention said drug or prodrug is a derivative of a medicament as defined hereinafter.

The present invention also relates to a method of diagnosing a disorder related to the presence of a molecular variant of the OCT1 gene or susceptibility to such a disorder comprising determining the presence of a polynucleotide or the gene of the invention in a sample from a subject.

In accordance with this embodiment of the present invention, the method of testing the status of a disorder or susceptibility to such a disorder can be effected by using a polynucleotide gene or nucleic acid of the invention, e.g., in the form of a Southern or Northern blot or *in situ* analysis. Said nucleic acid sequence may hybridize to a coding region of either of the genes or to a non-coding region, e.g. intron. In the case that a complementary sequence is employed in the method of the invention, said nucleic acid molecule can again be used in Northern blots. Additionally, said testing can be done in conjunction with an actual blocking, e.g., of the transcription of the gene and thus is expected to have therapeutic relevance. Furthermore, a primer or oligonucleotide can also be used for hybridizing to one of the above mentioned OCT1 gene or corresponding mRNAs. The nucleic acids used for hybridization can, of course, be conveniently labeled by incorporating or attaching, e.g., a radioactive or other marker. Such markers are well known in the art. The labeling of said nucleic acid molecules can be effected by conventional methods. Additionally, the presence or expression of variant OCT1 gene can be monitored by using a primer pair that specifically hybridizes to either of the corresponding nucleic acid sequences and by carrying out a PCR reaction according to standard procedures. Specific hybridization of the above mentioned probes or primers preferably occurs at stringent hybridization conditions. The term "stringent hybridization conditions" is well known in the art; see, for example, Sambrook et al., "Molecular Cloning, A Laboratory Manual" second ed., CSH Press, Cold Spring Harbor, 1989; "Nucleic Acid Hybridization, A Practical Approach", Hames and Higgins eds., IRL Press, Oxford, 1985. Furthermore, the mRNA, cRNA, cDNA or genomic DNA obtained from the subject may be sequenced to identify mutations which may be characteristic fingerprints of mutations in the polynucleotide or the gene of the invention. The present invention further comprises methods wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments as described above.

Further modifications of the above-mentioned embodiment of the invention can be easily devised by the person skilled in the art, without any undue experimentation from this disclosure; see, e.g., the examples. An additional embodiment of the present invention relates to a method wherein said determination is effected by employing an antibody of the invention or fragment thereof. The antibody used in the method of the invention may be labeled with detectable tags such as a histidine flags or a biotin molecule.

The invention relates to a method of diagnosing a disorder related to the presence of a molecular variant of an OCT1 gene or susceptibility to such a disorder comprising determining the presence of a polypeptide or the antibody of the invention in a sample from a subject.

In a preferred embodiment of the diagnostic method said disorder comprises side effects, or reduced activity of drug therapy, or non-activity of drug therapy as a result from aberrant serum and/or intracellular concentrations of compounds that are substrates of the transporter OCT1.

In another preferred embodiment of the present invention, the above described method is comprising DNA sequencing, hybridisation techniques, PCR based assays, fluorescent dye and quenching agent-based PCR assay (Taqman PCR detection system), RFLP-based techniques, single strand conformational polymorphism (SSCP), denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, DHPLC-based techniques, oligonucleotide extension assays (OLA), extension based assays (ARMS, (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), pyrosequencing, protein truncation assay (PTT), immunoassays, haplotype analysis, and solid phase hydridization (dot blot, reverse dot blot, chips).

Said techniques are very well known in the art.

Moreover, the invention relates to a method of detection of the polynucleotide or the gene of the invention in a sample comprising the steps of
(a) contacting the solid support described supra with the sample under conditions allowing interaction of the polynucleotide or the gene of the invention with the immobilized targets on a solid support; and
(b) determining the binding of said polynucleotide or said gene to said immobilized targets on a solid support.

The term "contacting" as referred to herein encompasses all techniques which enable a direct contact between the immobilized targets on the solid support and the polynucleotide or gene of the invention present in a sample. Preferably, contacting occurs in a liquid or gel or at least under humid atmosphere. The liquid or gel may be supplemented with a suitable buffer which allows or enhances interaction between the immobilized targets and the polynucleotides or genes of the invention present in the sample. Suitable liquids or gels for this purpose are well known in the art and are described in, e.g., Cheung, Nat. Genet. 21 (1999), 15-9. More preferably, electric fields are used to accelerate the contact between the immobilized target and the sample.

The term "conditions allowing interaction" refers, preferably, to those conditions under which a specific interaction takes place. Specificity of the interaction is, in principle, governed by ionic strength of the incubation liquid and temperature, electric fields or dependent on the agitation system used as disclosed for example in US 6,287,850. The person skilled in the art can adjust suitable conditions for detection by routine experimentation. Preferably, the term "conditions allowing interaction" refers to reactions where polynucleotides can be bound by ligases or via chemical or photochemical reactions. For detection methods including fluorescence, chemiluminescence, mass spectrometry, and also conductivity and electronic methods, can be used as described for example in Watson, Current opinion in Biotechnology 9 (1998), 609-614.

The invention also relates to an in vitro method for diagnosing a disease comprising the steps of the method described supra, wherein binding of said polynucleotide or gene to said immobilized targets on said solid support is indicative for the presence or the absence of said disease or a prevalence for said disease.

The invention furthermore relates to a diagnostic composition comprising the polynucleotide, the gene, the vector, the polypeptide or the antibody of the invention.

In addition, the invention relates to a pharmaceutical composition comprising the polynucleotide, the gene, the vector, the polypeptide or the antibody of the invention.

These pharmaceutical compositions comprising, e.g., the antibody may conveniently be administered by any of the routes conventionally used for drug administration, for instance, orally, topically, parenterally or by inhalation. Acceptable salts comprise acetate, methylester, HCI, sulfate, chloride and the like. The compounds may be administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable character or diluent is dictated by the amount of active ingredient with which it is to be combined, the route of administration and other well-known variables. The carrier(s) must be "acceptable" in the sense of being compatible with the other ingredients of the formulation and not deleterious to the recipient thereof. The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

Furthermore, the use of pharmaceutical compositions which comprise antisense-oligonucleotides which specifically hybridize to RNA encoding mutated versions of the polynucleotitde or gene according to the invention or which comprise antibodies specifically recognizing a mutated polypeptide of the invention but not or not substantially the functional wild-type form is conceivable in cases in which the concentration of the mutated form in the cells should be reduced.

Thanks to the present invention the particular drug selection, dosage regimen and corresponding patients to be treated can be determined in accordance with the present invention. The dosing recommendations will be indicated in product labeling by allowing the prescriber to anticipate dose adjustments depending on the considered patient group, with information that avoids prescribing the wrong drug to the wrong patients at the wrong dose.

In another embodiment the present invention relates to the use of the polynucleotide, the gene, the vector, the polypeptide, the polynucleotides having the polynucleotide sequences of SEQ ID NO: 18, 19, 20, 21, 22, 23, 24, 25, 26 or 27, the polypeptide of SEQ ID NO: 34 or 35, or the antibody of the invention for the preparation of a diagnostic composition for diagnosing a disease.

In a further embodiment the present invention relates to the use of the polynucleotide, the gene, the vector, the polypeptide, the polynucleotides having the polynucleotide sequences of SEQ ID NO: 18, 19, 20, 21, 22, 23, 24, 25, 26 or 27, the polypeptide of SEQ ID NO: 34 or 35 , or the antibody of the invention for the preparation of a pharmaceutical composition for treating a disease.

A gene encoding a functional and expressible polypeptide of the invention can be introduced into the cells which in turn produce the protein of interest. Gene therapy, which is based on introducing therapeutic genes into cells by *ex-vivo* or *in-vivo* techniques is one of the most important applications of gene transfer. Suitable vectors and methods for *in-vitro* or *in-vivo* gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. The gene may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.

As is evident from the above, it is preferred that in the use of the invention the nucleic acid sequence is operatively linked to regulatory elements allowing for the expression and/or targeting of the polypeptides of the invention to specific cells. Suitable gene delivery systems that can be employed in accordance with the invention may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as that described by Williams (Proc. Natl. Acad. Sci. USA 88 (1991), 2726-2729). Standard methods for transfecting cells with recombinant DNA are well known to those skilled in the art of molecular biology, see, e.g., WO 94/29469; see also supra. Gene therapy may be carried out by directly administering the recombinant DNA molecule or vector of the invention to a patient or by transfecting cells with the polynucleotide or vector of the invention ex vivo and infusing the transfected cells into the patient.

In a more preferred embodiment of the use of the present invention said disease comprises side effects, or reduced activity, or non-activity of drug therapy as a result from aberrant serum and/or intracellular concentrations of compounds that are substrates of the transporter OCT1.

Finally, the present invention relates to a diagnostic kit for detection of a single nucleotide polymorphism comprising the polynucleotide, the gene, the vector, the polypeptide, the antibody, the host cell, the transgenic non-human animal or the solid support of the invention.

The kit of the invention may contain further ingredients such as selection markers and components for selective media suitable for the generation of transgenic cells and animals. The kit of the invention can be used for carrying out a method of the invention and could be, inter alia, employed in a variety of applications, e.g., in the diagnostic field or as research tool. The parts of the kit of the invention can be packaged individually in vials or other appropriate means depending on the respective ingredient or in combination in suitable containers or multicontainer units. Manufacture of the kit follows preferably standard procedures which are known to the person skilled in the art. The kit may be used for methods for detecting expression of a mutant form of the polypeptides, genes or polynucleotides in accordance with any one of the above-described methods of the invention, employing, for example, immunoassay techniques such as radioimmunoassay or enzymeimmunoassay or preferably nucleic acid hybridization and/or amplification techniques such as those described herein before and in the Examples as well as pharmacokinetic studies when using non-human transgenic animals of the invention.

The nucleic acid and amino acid sequences referred to herein are shown in the following Tables1 to 4.

**Table 1: New OCT1 nucleotide change**

| **SEQ ID NOS** | **Mutant sequence (5'->3')** | **Position of the mutation** | **Reference sequence** |
|---|---|---|---|
| 1 | AAATGGCCAaTTGAATTCA | 107155 | GI:9581607 |
| 2 | TACCCTTTCaCCAGCATGT | 107265 | GI:9581607 |
| 3 | GCATGTCAGcCTGCTGAGC | 107278 | GI:9581607 |
| 4 | CTGAGCCAGtGCTGTGGCT | 109130 | GI:9581607 |
| 5 | CCTTGGCCAGcGCAGGCGCTA | 109211 | GI:9581607 |
| 6 | TCCCACCTGtCCTCCATGT | 119220 | GI:9581607 |
| 7 | TCTCCCTCCtTCCTAGATG | 123551 | GI:9581607 |
| 8 | GACCGCGTGaGCCGCATCT | 126806 | GI:9581607 |
| 9 | TGTTGGCGGcGGCAGCCTG | 126846 | GI:9581607 |
| 10 | TGCCTCGT**CA**TTTTTATC | 126863 to 126865 | GI:9581607 |
| 11 | TCCCAGGCAgTCGAAGTGT | 126922 | GI:9581607 |
| 12 | CATCATTTCtCAGGCAATC | 126915 | GI:9581607 |
| 13 | GGTGAGTGCgTGGAACAGG | 130672 | GI:9581607 |
| 14 | AGGAACCTCaGAGTGATGG | 141819 | GI:9581607 |
| 15 | ATTGGCTGTaCTCTAATGG | 142951 | GI:9581607 |
| 16 | CCTTCTTTTtCAGCTCGGC | 141961 | GI:9581607 |
| 17 | TGAAGCGGTaTTGGGCCTG | 142993 | GI:9581607 |

**Table 2: New OCT1 amino acid change**

| **SEQ ID NOS** | **Mutant sequence (5'->3')** | **Position of the mutation** | **Reference sequence** |
|---|---|---|---|
| 28 | AELSQ**C**CGWSP | R61C | GI:2511670 |
| 29 | AFLGQ**R**RRYEV | C88R | GI:2511670 |
| 30 | TIDRV**S**RIYPM | G401S | GI:2511670 |
| 31 | SNLLA**A**AACLV | G414A | GI:2511670 |
| 32 | AACLVIFISPD | M420del | GI:2511670 |
| 33 | FVRNL**R**VMVCS | G465R | GI:2511670 |

**Table 3: OCT1 nucleotide change also listed in the databases**

| **SEQ ID NOS** | **Mutant sequence (5'->3')** | **Position of the mutation** | **Reference sequence** |
|---|---|---|---|
| 18 | CACACATGGgTCTGTGCTT | 117075 | GI:9581607 |
| 19 | TGACCAGTTaGAATTAACT | 117318 | GI:9581607 |
| 20 | AGCCCCAACaTGGGGAGGG | 123136 | GI:9581607 |
| 21 | TGGTAAG**TT**GTCTGCTT | 126888 to 126895 | GI:9581607 |
| 22 | CTGCCAGAGcCCTGGGGTG | 109105 | GI:9581607 |
| 23 | CGGGCTTCTTgTTTGGCTCTC | 552 | GI:2511669 |
| 24 | CCCCATGGCCgTGTCAAATTT | 126827 | GI:9581607 |
| 25 | CCACCAGCTgTAATAGTCC | 130458 | GI:9581607 |
| 26 | TTTTGCAGCTtGGCAGTGGGC | 141967 | GI:9581607 |
| 27 | TTAACTCCAAtTTTTAATTTT | 145509 | GI:9581607 |

**Table 4: OCT1 amino acid changes also listed in the databases**

| **SEQ ID NOS** | **Mutant sequence (5'->3')** | **Position of the mutation** | **reference sequence** |
|---|---|---|---|
| 34 | LNAGF**L**FGSLG | F160L | GI:2511670 |
| 35 | IYPMA**V**SNLLA | M408V | GI:2511670 |

The figures illustrate the invention:

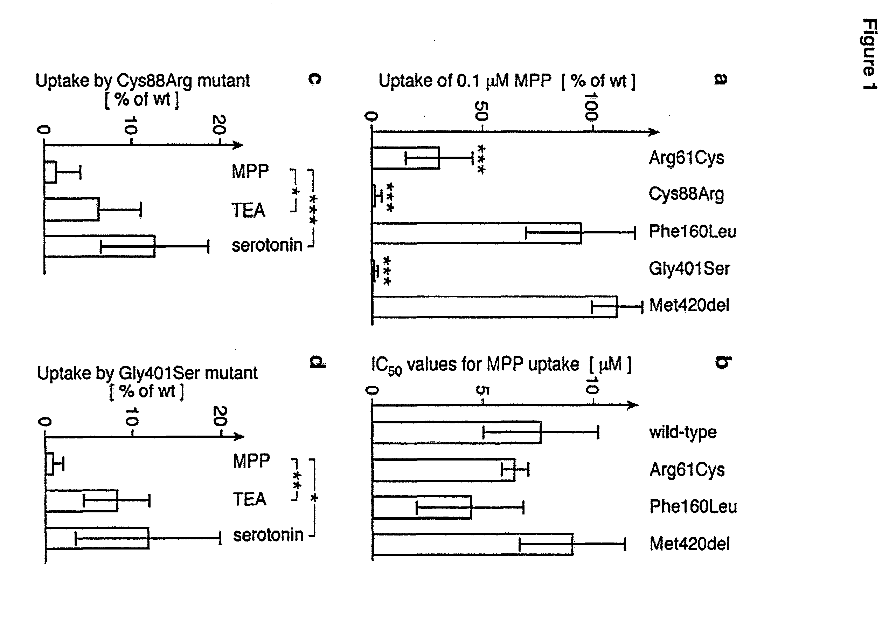

**FIG. 1****:** Functional characterization of missense mutations of OCT1. OCT1 wt and OCT1 mutants were expressed in *Xenopus* oocytes and analyzed side-by-side. Cyanine863-inhibitable uptake of radioactively labeled organic cations was measured over 30 min. a) Uptake of 0.1 *µ* M [³H]MPP by different mutants in comparison to OCT1. Mean uptake and SD of 3-8 individual measurements are shown. ***P<0.001 for difference compared to OCT1 wt. In different batches of oocytes, the cyanine863-inhibited uptake of 0.1 *µ*M [³H]MPP expressed by OCT1 wt varied between 0.9 and 1.8 pmol x oocyte⁻¹ x 30 min⁻¹. The cyanine863-inhibited uptake in water-injected control oocytes was always smaller than 0.02 pmol x oocyte⁻¹ x 30 min⁻¹. b) Concentration dependence of MPP uptake by OCT1 wt and OCT1 mutants. MPP uptake was measured at 9 different MPP concentrations and K_{0.5} values were determined by fitting the Hill equation to the data. Mean K_{0.5} ± SD of 3 (mutants) or 6 (wt) independent experiments are shown. c,d) Uptake of MPP, TEA and serotonin by Cys88Arg (c) and Gly401Ser (d) compared to OCT1 wt. Uptake of 0.1 *µ*M MPP, 10 *µ*M TEA and 1 *µ*M serotonin were measured side-by-side using the same oocyte batch and substrates. Mean values ± SD of three experiments are presented. *P<0.05, **P<0.01, ***P<0.001.

The invention will now be described by reference to the following biological Examples which are merely illustrative and are not constructed as a limitation of the scope of the present invention.

### Example 1: Identification of Variations of the Human Organic Cation Transporter OCT1

A systematic screening for genetic variants in the gene encoding the polyspecific cation transporter OCT1 (SLC22A1) was performed in Caucasian individuals. For that, blood was obtained from 57 healthy (based on medical history, clinical investigations, and routine laboratory parameters) Caucasians (mean(SD) age 43.1 (17.6), 40 male, 17 female) after ethical approval and written informed consent. A second group of 190 healthy Caucasians (mean(SD) age 38.8(11.3), 129 male, 61 female) was collected according to the same medical, clinical, laboratory, and ethical principles to establish the population frequency of selected genotypes. DNA was isolated using the Qiamp system (Qiagen, Hilden, Germany) on a Qiagen 9604 robot. Identification of the polymorphism was done by sequencing, using oligonucleotide primers for amplification of specific OCT1 gene (Genbank accession number Gl:9581607) fragments (11 exons and 2 kb of the promoter region) were designed to span the complete exons plus at least 50 bp of each adjacent intron. The DNA sequences of purified PCR fragments were obtained on a ABl3700 capillary sequencer (ABI, Weiterstadt, Germany) and assembled using the phredPhrap software (University of Washington).

The sequences of the primers that were used to specifically amplify OCT1 gene fragments are listed in Table 5.

25 nucleotide variations were detected by sequencing all 11 exons of OCT1 including at least 50 bp of the adjacent introns and 2 kb of the promoter. For 16 variations the population frequency was established by analyzing additional 190 Caucasians. The positions of the variations and their genotype frequencies are listed in Table 6. Three variations were in the promoter region, 10 in the coding region and 12 in the introns. Eight of these variations resulted in an amino acid exchange and several variations were linked in all investigated subjects. Mutation Met408Val was linked with a deletion of TGGTAAGT at position 17939, SNP 33012G>T in intron 9 with the silent variation 34044G>A in exon 10, the -1795G>A substitution in the promoter with the silent 156T>C variation in exon 1, and 10270C>T in intron 2 with 14602C>T substitution in intron 5.

**Table 5: Nucleotide sequence and localization of primers for fragment generation and sequencing.**

| **Localization** | **Primer** | **Sequence [5'-3'a** | **Fraqment size** |
|---|---|---|---|
| **Exon 1** | OA-E1f | aacgatttgatcagatggccacg | |
| | OA-E1 r | ccagacacccacgaactgc | 758bp |
| **Exon 2** | OA-E2f3 | aaacagcccagggataccgag | |
| | OA-E2r1 | cccacagtatcccaaagcagg | 392bp |
| **Exon 3** | OA-E3f1 | ctccgactgtgacccttgg | |
| | OA-E3r2 | aactggtgccccgcaagctc | 366bp |
| **Exon 4** | OA-E4f | ccgagcttctgaacgcacg | |
| | OA-E4r | actggtccctcgagaggac | 327bp |
| **Exon 5** | OA-E5f | atcctcttgagggattacagcc | |
| | OA-E5r | ccccagacgaatctgcacc | 309bp |
| **Exon 6** | OA-E6f | atgggtgtgaagcacggtgg | |
| | OA-E6r | gagtattccactgtctctaatctatagc | 297bp |
| **Exon 7** | OA-E7f1 | tttcttcagtctctgactcatgcc | |
| | OA-E7r | aaaaaactttgtagacaaaggtagcacc | 396bp |
| **Exon 8** | OA-E8f | aaaagttagataagacaaacttccaggc | |
| | OA-E8r | ggctgcatctttaggaagcacc | 339bp |
| **Exon 9** | OA-E9f | aagctgcaggtattggcattgtac | |
| | OA-E9r | agccagaagacatcccaagagc | 386bp |
| **Exon 10** | OA-E10f1 | aatgaaggcaatgtttcctttacgtactc | |
| | OA-E10r | aagacatacaaatatctgtaaagctctcc | 452bp |
| **Exon 11** | OA-E11f1 | aaacaggctataagctcgaatggg | |
| | OA-E11 r | cctagatcgaatgcacaggtgg | 487bp |
| **Promoter** | OA-P1f1 | tacacacacacaaatgaagaggtgg | |
| | OA-P1r1 | tggtttgaaatcagtttgctgtccaag | 537bp |
| **Promoter** | OA-P2f | ggccctaccaaactgcaaagc | |
| | OA-P2r2 | atggtatgaggcaagtattgggtg | 568bp |
| **Promoter** | OA-P3f | cttactcacctcacgtgtagatctg | |
| | OA-P3r | cttaagtatgactttgctaaataggctgtc | 321bp |
| **Promoter** | OA-P4f | cttcccttcttgtgtcagtagc | |
| | OA-P4r | gagctaccattataacatgtagtgatgac | 565bp |
| **Promoter** | OA-P5f | cctctccctttcgatgctcc | |
| | OA-P5r | caaggttttcttgaagcacttacatgc | 718bp |

**Table 6: Localization, function, and allelic frequency of hereditary polymorphisms in the human OCT1 gene**

| **Genetic** | **Variation** | | **Cellular** | **Genotype Frequency [%]** | | | |
|---|---|---|---|---|---|---|---|
| **Localization** | **Nucleotide^{a}** | **Amino acid** | **Localization** | **N** | **Wt** | **Het** | **Mut** |
| Promoter | -1795G>A | | | 55 ° | 74.5 | 23.7 | 1.8 |
| Promoter | -1685G>A | | | 54 ° | 98.1 | 1.9 | 0 |
| Promoter | -1672G>C | | | 54° | 98.1 | 1.9 | 0 |
| Exon 1 | 156T>C* | silent | | 243 ^{d} | 60.0 | 34.2 | 5.8 |
| Exon 1 | 181C>T | Arg61 Cys^{b} | large extrac. loop | 243 ° | 83.2 | 15.6 | 1.2 |
| Exon 1 | 262T>C | Cys88Arg^{b} | large extrac. loop | 243 ^{d} | 98.8 | 1.2 | 0 |
| Exon 2 | 8237C>G* | Phe160Leu^{b} | 2. TMD | 241 ^{d} | 61.4 | 34.0 | 4.6 |
| Exon 7 | 17857G>A | Gly401Ser^{b} | MSF-signature^{d} | 217^{d} | 93.5 | 6.5 | 0 |
| Exon 7 | 17878A>G* | Met408Va1 | 9. TMD | 232 ^{d} | 17.7 | 45.2 | 37.1 |
| Exon 7 | 17897G>C | Gly414A1a | 9. TMD | 232 ^{d} | 99.6 | 0.4 | 0 |
| Exon 7 | 17914delATG | Met420de1^{b} | 9. TMD | 232^{d} | 71.1 | 26.3 | 2.6 |
| Exon 9 | 32870G>A | Gly465Arg | 5. intrac. loop | 236 ^{d} | 97.0 | 3.0 | 0 |
| Exon 10 | 34044G>A | silent | | 56^{c} | 94.6 | 3.6 | 1.8 |
| Intron 1 | 8126T>G | | | 240 ^{d} | 83.3 | 16.3 | 0.4 |
| lntron 2 | 8369G>A | | | 240^{d} | 89.6 | 10.4 | 0 |
| Intron 2 | 10270C>T | | | 57^{c} | 77.1 | 21.1 | 1.8 |
| Intron 5 | 14602C>T | | | 54^{c} | 79.6 | 18.5 | 1.9 |
| Intron 7 | 17939deITGGTAAGT | | | 233^{d} | 18.0 | 45.1 | 36.9 |
| Intron 7 | 17966C>T | | | 236^{d} | 76.3 | 21.2 | 2.5 |
| Intron 7 | 17972A>G | | | 237 ^{d} | 98.7 | 1.3 | 0 |
| Intron 8 | 21723A>G | | | 53 ^{c} | 98.1 | 1.9 | 0 |
| Intron 9* | 33017C>T* | | | 233^{d} | 36.9 | 46.4 | 16.7 |
| Intron 9 | 33012G>T | | | 235 ^{d} | 97.9 | 1.7 | 0.4 |
| Intron 9 | 34002G>A | | | 56^{c} | 98.2 | 1.8 | 0 |
| Intron 10 | 36560C>T* | | | 57^{c} | 59.6 | 35.1 | 5.3 |

| | | | | | | | |
|---|---|---|---|---|---|---|---|
| ^{a} The genomic sequence with the GenBank Accession number Gl:9581607 is used as reference sequence, Gl:4506998 gives the cDNA sequence of OCT1. The incorrect annotation of exons 3 and 4 in Gl 9581607 did not affect our analysis. For nucleotide numbering the A of the ATG start codon (position 108950-108952 of Gl: 9581607) is denoted +1. The first nucleotode after the A is denoted +2, the first nucleotide before the A of ATG is denoted ―1. * indicates mutations that are also listed in the SNP database of National Center of Biotechnology Information but had not been verified. ^{b} analyzed for function. ^{d}lntracellular loop between TMD 8 and 9 that is conserved in the superfamily of major solute facilitators. ^{c} 57 subjects investigated. ^{d} 247 subjects investigated. TMD, transmembrane domain. N, number of successfully genotyped subjects; Wt, homozygous for reference genotype (Gl:9581607 andGl:251169); Het, heterozygous genotype; Mut, homozygous for mutation. | | | | | | | |

### Example 2: Functional Consequences of Variations of the Human Organic Cation Transporter OCT1

To functionally characterize OCT1 variants by transport measurements, site directed mutagenesis was used to generate plasmids for recombinant expression of OCT1 and OCT1 variants in xenopus oocytes.

Altogether, five of the missense mutations were characterized by transport measurements. The point mutations in the predicted 9^{th} transmembrane domain (TMD) and 5^{th} intracellular loop were excluded since point mutations in OCT1 from rat suggested that these mutations do not lead to functional changes (unpublished data). The characterized mutations are localized in the large extracellular loop (Arg61 Cys, Cys88Arg), in TMD 2 (Phe160Leu), in the highly conserved short intracellular loop between TMD 8 and 9 (Koepsell, J. Membr. Biol. 167 (1999), 103-117, Gorboulev, DNA Cell Biol. 16 (1999), 871-881) (Gly401Ser), and in TMD 9 (Met420del).

The point mutations were introduced into wild-type (wt) OCT1 by PCR using the overlap extension method, and the amplificates with the mutations were cloned into OCT1 wt as described by Gorboulev *et al.* (Gorboulev, DNA Cell Biol. 16 (1999), 871-881). The presence of OCT1 mutations was verified by DNA sequencing, and or expression in *Xenopus laevis* oocytes, OCT1 wt and OCT1 mutants were cloned into appropriate vector systems (Arndt, Am J. Physiol. Renal Physiol. 281 (2001), F454-468). For the expression in *Xenopus laevis* oocytes, the pOG1 vector containing OCT1 wt and mutants was linearized with *Not* I*,* and sense cRNAs were transcribed as described (Arndt, Am J. Physiol. Renal Physiol. 281 (2001), F454-468). After defolliculation, the oocytes were injected with 10 ng/oocycte of the respective cRNAs. After 3 days of incubation at 16°C, uptake measurements were performed with [³H]MPP, [¹⁴C]TEA and [³H]serotonin. Oocytes were incubated for 30 min with the indicated substrate concentrations in the absence or presence of 100 *µ*M of the inhibitor cyanine863. The mutants were compared with the OCT1 wt in side-by-side experiments using oocytes from the same batch (Arndt, Am J. Physiol. Renal Physiol. 281 (2001), F454-468). Each data point corresponded to 8-10 oocytes. K₀.₅ values were estimated by fitting the Hill equation to the data. Mean values ± SD are presented. Significance of differences was tested by unpaired Student t-tests.

Fig.1a shows that the uptake of 0.1 *µ*M [³H]MPP by mutant Arg61 Cys was reduced by 70% whereas MPP uptake by mutants Cys88Arg and Gly401 Ser were reduced by more than 98%. At variance, the uptake of 0.1 *µ*M [³H]MPP by mutants Phe160Leu and Met420del were not significantly different from OCT1 wt and showed half maximal concentration for substrate activation (K₀.₅) values (Fig.1b) and maximal expressed transport rates (data not shown) that were identical to wild-type. For the Phe160Leu mutant a similar K₀.₅ value (Fig.1b) and a 32 ± 16% (n=3) reduced Vₘₐₓ value compared to OCT1 wt was observed. To determine whether the mutations affect substrate selectivity and whether the Cy88Arg and Gly401Ser mutants may transport other cations better than MPP, we measured the uptake of 0.1 *µ*M [³H]MPP, 10 *µ*M [¹⁴C]TEA and 1 *µ*M [³H]serotonin in parallel and in comparison with OCT1 wt. For the mutants Arg61Cys, Phe160Leu and Met420del no significant changes in substrate selectivity were detected in three independent experiments (data not shown). At variance, significant changes in substrate specificity were observed for the Cys88Arg and Gly401Ser mutants (Fig.1 c,d). In these mutants compared to wt, the uptake of 10 *µ*M TEA and 1 *µ*M serotonin was significantly less reduced than the uptake of 0.1 *µ*M MPP.

### Example 3: Correlation of Variations of the Human Organic Cation Transporter OCT1 with Drug-Induced Cholestasis

Human OCT1 plays a major role in hepatic uptake of cations (Briz, Mol. Pharmacol. 61 (2002), 853-860; Dresser, J. Pharm. Sci. 90 (2001), 397-421; Gorboulev, DNA Cell Biol. 16 (1999), 871-881, Koepsell, J. Membr. Biol. 167 (1999), 103-117; van Montfoortl, J. Pharmacol. Exp. Ther. 298 (2001), 110-115), participates in the removal of neurotransmitters from the interstitial space ( Chen, J. Neurosci. 21 (2001), 6348-6361), mediates cellular release of acetylcholine (Wessler, Br. J. Pharmacol. 134 (2001), 951-956), and participates in the excretion of prostaglandins (Kimura, J. Pharmacol. Exp. Ther. 301 (2002), 293-298). Because of this direct action on various compounds including physiological substrates (acetylcholine, serotonin) as well as drugs, functionally important variations of OCT1 may be the cause of or attribute to deviant drug action. Therefore, OCT1 variants may be associated with the occurrence of reduced activity of drugs or -vive versa- with side effects of drugs in individual patients that are carriers of OCT1 variants. As a consequence of altered pharmacokinetics, an enhanced duration and intensity of drug with implication for drug efficacy, safety, and tolerability can be anticipated in carriers of functional OCT1 variants. Table 7 shows the results of analysis of OCT1 variants in patients that suffered from 'drug-induced-cholestasis. The frequency of OCT1 variants in this patient cohort was compared to with a control group, for which drug-induced cholestasis (DIC) was not observed. Most striking is the significant association between the Met408Val SNP (SEQ ID NO: 24, 35) and the linked 8 bp deletion (SEQ ID NO: 21) and the occurrence of drug-induced cholestasis. These polymorphisms occur only in 30% of the normal population, but in patients suffering from drug-induced side effects, the frequency of the polymorphism is increased to more than 70%. Thus, the diagnosis of these OCT1 polymorphisms is useful to predict with statistical significance a greatly increased individual risk to encounter side effects of drug therapy. Thus, OCT1 genotyping can serve as a useful tool to predict and thereby control and avoid undesired side effects of drug therapy. Another example for the association of OCT1 polymorphisms with a clinical phenotype is a significant correlation of the genetic variant Gly401Ser of the OCT1 gene with patients suffering from hepatic side effects as a consequence of drug therapy compared to controls (Table 8).

**Table 7: Analysis of OCT1 polymorphism and drug-induced cholestasis (DIC)**

| | | | | **Controls** | **DIC** |
|---|---|---|---|---|---|
| | | **A** | N | 43 | 1 |
| | | | % | 17,10% | 9,10% |
| **Met408Val** | | **AG** | N | 114 | 2 |
| | | | % | 45,40% | 18,20% |
| | | **G** | N | 94 | 8 |
| | | | % | 37,50% | 72,70% |
| | Total | | N | 251 | 11 |
| | | | % | 100,00% | 100,00% |

Subjects were grouped according to the SNP-genotype in order to explore the influence of the Met408Val polymorphism of the OCT1 gene on the occurrence of drug-induced cholestasis DIC) Significant differences could be observed for the allelic frequency of A and G carriers between controls and DIC patients (p=0.041). Significant differences have also been observed for the linked variant 17939delTGGTAAGT.

**Table 8: Analysis of OCT1 polymorphisms and drug-induced hepatotoxic side effects**

| | | | **Controls** | **HepTox** |
|---|---|---|---|---|
| **Gly401Se** | **AG** | N | 14 | 5 |
| | | % | 6,20% | 20,80% |
| | **G** | N | 211 | 19 |
| | | % | 93,80% | 79,20% |
| Total | | N | 225 | 24 |
| | | % | 100,00% | 100,00% |

Subjects were grouped according to SNP-genotype in order to explore the influence of the Gly401Val polymorphism of the OCT1 gene on the occurrence of hepatotoxic side effects. The distribution of genotypes between the groups was statistically significant (p=0.025, Fisher's exact test, 2-sided), and reflects an association of this polymorphism on the development of hepatotoxicity. A significant difference could also be observed for the allelic frequency of A and G carriers between the two groups (p=0.012).

### Example 4: Linkage of Polymorphisms defines Alleles and Haplotypes of the Human Organic Cation Transporter OCT1, which are associated with Drug-Induced Cholestasis

Defined genetic variations of genes can directly be associated with corresponding phenotypes in some cases. In many other cases, however, it is known that the determination of haplotypes, i.e. the knowledge of the combination of defined alleles, is more predictive of a phenotype than the determination of single polymorphisms. Therefore, it is important to determine and assign OCT1 alleles to linkage groups and alleles. This information is important for subsequent haplotyping and for identification of functional and variant alleles. The analysis of the identified SNPs in different individuals reveals that some OCT1 SNPs ocurr linked to each other. This defines OCT1 alleles: The Met408Val Polymorphism was found to be linked with a deletion of TGGTAAGT at position 17939, SNP 33012G>T in intron 9 is linked with the synonymous polymorphism 34044G>A in exon 10, the -1795G>A substitution in the promoter with the synonymous 156T>C variation in exon 1, and 10270C>T in intron 2 with 14602C>T substitution in intron 5. Obviously, other so far undiscovered- SNPs can also be present in the larger region of these defined alleles, but the information described herewith is sufficient to unambiguously identify the alleles and allele clusters.

The Met408-Val Polymorphism, which has been identified to be associated with drug-induced cholestasis (see Example 3), belongs to an allele that differs from the OCT1 wild-type sequence with at least 2 positions: Thus, a diagnostic assay for the prediction of drug-induced cholestasis is not limited to the SNPs, but rather consists of the determination of alleles, that are defined by the presence or absence of these polymorphisms.

In view of the above, it will be appreciated that the invention also encompasses the following items:
1. A polynucleotide comprising a polynucleotide selected from the group consisting of:
   (a) a polynucleotide having the nucleic acid sequence of SEQ ID NO: 4, 5, 6, 7, 8, 9, 10, 11, 12, 13, 14, 15, 16 or 17;
   (b) a polynucleotide encoding a polypeptide having the amino acid sequence of SEQ ID NO: 28, 29, 30, 31, 32 or 33;
   (c) a polynucleotide having a nucleic acid sequence with at least 70%, preferably at least 75%, at least 80%, at least 85%, at least 90% or at least 95% sequence identity to an OCT1 gene, wherein said polynucleotide is having a nucleotide exchange or a nucleotide deletion of at least one nucleotide at a position 109130, 109211, 119220, 123551, 126806, 126846, 126863 to 126865, 126922, 126915, 130672, 141819, 142951, 141961 or 142993 of the OCT1 gene (GenBank Accession No: Gl:9581607).
   (d) a polynucleotide capable of hybridizing to an OCT1 gene, wherein said polynucleotide is having a substitution of at least one nucleotide at a position corresponding to position 109130, 109211, 119220, 123551, 126806, 126846, 126922, 126915, 130672, 141819, 142951, 141961 or 142993 of the OCT1 gene (GenBank Accession No: G1:9581607) or a deletion of three nucleotides at a position corresponding to position 126863 to 126865 of the OCT1 gene (GenBank Accession No: Gl:9581607);
   (e) a polynucleotide capable of hybridizing to an OCT1 gene, wherein said polynucleotide is having an A at a position corresponding to position 126806, 141819, 142951 or 142993 of the OCT1 gene (GenBank Accession No: Gl: 9581607), a C at a position corresponding to position 109211 or 126846 of the OCT1 gene (GenBank Accession No: Gl: 9581607), a G at a position corresponding to position 126922 or 130672 of the OCT1 gene (GenBank Accession No: Gl: 9581607), a T at a position corresponding to position 109130, 119220, 123551, 126915 or 141961 of the OCT1 gene (GenBank Accession No: Gl: 9581607) or an ATG deletion at a position corresponding to position 126863 to 126865 of the OCT1 gene (GenBank Accession No: Gl:9581607);
   (f) a polynucleotide encoding an OCT1 polypeptide or fragment thereof, wherein said polypeptide comprises an amino acid substitution at position 61, 88, 401, 414 or 465 of the OCT1 polypeptide (GenBank Accession No:Gl:2511670); and
   (g) a polynucleotide encoding an OCT1 polypeptide or fragment thereof, wherein said polypeptide comprises an amino acid substitution of R to C at position 61, an amino acid substitution of C to R at position 88, an amino acid substitution of G to S at position 401, an amino acid substitution of G to A at position 414, an amino acid deletion of M at position 420 or an amino acid substitution of G to R at position 465 of the OCT1 polypeptide (GenBank Accession No: Gl:2511670).
2. The polynucleotide of item 1, wherein said polynucleotide is associated with side effects, or reduced activity of drug-therapy, or non-activity of drug therapy as a result from aberrant serum and/or intracellular concentrations of compounds that are substrates of the transporter OCT1.
3. The polynucleotide of item 1 or 2 which is DNA or RNA.
4. A gene comprising the polynucleotide of item 1 or 2.
5. The gene of item 4, wherein a nucleotide deletion and/or substitution results in altered expression of the variant gene compared to the corresponding wild type gene.
6. A vector comprising a polynucleotide of any one of item 1 to 3 or the gene of item 4 or 5.
7. The vector of item 6, wherein the polynucleotide is operatively linked to expression control sequences allowing expression in prokaryotic or eukaryotic cells or isolated fractions thereof.
8. A host cell genetically engineered with the polynucleotide of any one of item 1 to 3, the gene of item 4 or 5 or the vector of item 6 or 7.
9. A method for producing a molecular variant OCT1 polypeptide or fragment thereof comprising
   (a) culturing the host cell of item 8; and
   (b) recovering said protein or fragment from the culture.
10. A method for producing cells capable of expressing a molecular variant OCT1 polypeptide comprising genetically engineering cells with the polynucleotide of any one of item 1 to 3, the gene item 4 or 5 or the vector of item 6 or 7.
11. A polypeptide or fragment thereof encoded by the polynucleotide of any one of item 1 to 3, the gene of item 4 or 5 or obtainable by the method of item 9 or from cells produced by the method of item 10.
12. An antibody which binds specifically to the polypeptide of item 11.
13. The antibody of item 12 which specifically recognizes an epitope containing one or more amino acid substitution(s) resulting from a nucleotide exchange as defined in item 1 or 5.
14. The antibody of item 12 or 13 which is monoclonal or polyclonal.
15. A transgenic non-human animal comprising at least one polynucleotide of any one of item 1 to 3, the gene of item 4 or 5 or the vector of item 6 or 7.
16. The transgenic non-human animal of item 15 which is a mouse, a rat or a zebrafish.
17. A solid support comprising one or a plurality of the polynucleotide of any one of item 1 to 3, the gene of item 4 or 5, the vector of item 6 or 7, the polypeptide of item 11, the antibody of item 12 or 13 or the host cell of item 8 in immobilized form.
18. The solid support of item 17, wherein said solid support is a membrane, a glass-or polypropylene- or silicon-chip, are oligonucleotide-conjugated beads or a bead array, which is assembled on an optical filter substrate.
19. An in vitro method for identifying a single nucleotide polymorphism said method comprising the steps of:
   (a) isolating a polynucleotide of any one item 1 to 3 or the gene of item 4 or 5 from a plurality of subgroups of individuals, wherein one subgroup has no prevalence for an OCT1 associated disease and at least one or more further subgroup(s) do have prevalence for an OCT1 associated disease; and
   (b) identifying a single nucleotide polymorphism by comparing the nucleic acid sequence of said polynucleotide or said gene of said one subgroup having no prevalence for an OCT1 associated disease with said at least one or more further subgroup(s) having a prevalence for an OCT1 associated disease.
20. A method for identifying and obtaining a pro-drug or a drug capable of modulating the activity of a molecular variant of an OCT1 polypeptide comprising the steps of:
   (a) contacting the polypeptide of item 11, the solid support of item 17 or 18, a cell expressing a molecular variant gene comprising a polynucleotide of any one of item 1 to 3, the gene of item 4 or 5 or the vector of item 6 or 7 in the presence of components capable of providing a detectable signal in response to drug activity with a compound to be screened for pro-drug or drug activity; and
   (b) detecting the presence or absence of a signal or increase or decrease of a signal generated from the pro-drug or the drug activity, wherein the absence, presence, increase or decrease of the signal is indicative for a putative pro-drug or drug.
21. A method for identifying and obtaining an inhibitor of the activity of a molecular variant of an OCT1 polypeptide comprising the steps of:
   (a) contacting the protein of item 11, the solid support of item 17 or 18 or a cell expressing a molecular variant gene comprising a polynucleotide of any one of items 1 to 3 or the gene of item 4 or 5 or the vector of item 6 or 7 in the presence of components capable of providing a detectable signal in response to drug activity with a compound to be screened for inhibiting activity; and
   (b) detecting the presence or absence of a signal or increase or decrease of a signal generated from the inhibiting activity, wherein the absence or decrease of the signal is indicative for a putative inhibitor.
22. The method of item 20 or 21, wherein said cell is a cell of item 8, obtained by the method of item 10 or can be obtained by the transgenic non-human animal of item 15 or 16.
23. A method of identifying and obtaining a pro-drug or drug capable of modulating the activity of a molecular variant of an OCT1 polypeptide comprising the steps of:
   (a) contacting the host cell of item 8, the cell obtained by the method of item 10, the polypeptide of item 11 or the solid support of item 17 or 18 with the first molecule known to be bound by an OCT1 polypeptide to form a first complex of said polypeptide and said first molecule;
   (b) contacting said first complex with a compound to be screened, and
   (c) measuring whether said compound displaces said first molecule from said first complex.
24. A method of identifying and obtaining an inhibitor capable of modulating the activity of a molecular variant of an OCT1 polypeptide comprising the steps of:
   (a) contacting the host cell of item 8, the cell obtained by the method of
      item 10, the protein of item 11 or the solid support of item 17 or 18 with the first molecule known to be bound by an OCT1 polypeptide to form a first complex of said polypeptide and said first molecule;
   (b) contacting said first complex with a compound to be screened, and
   (c) measuring whether said compound displaces said first molecule from said first complex.
25. The method of item 23 or 24, wherein said measuring step comprises measuring the formation of a second complex of said polypeptide and said compound.
26. The method of any one of item 23 to 25, wherein said measuring step comprises measuring the amount of said first molecule that is not bound to said polypeptide.
27. The method of any one of item 23 to 26, wherein said first molecule is labeled.
28. A method for the production of a pharmaceutical composition comprising the steps of the method of any one of item 20 to 27; and the further step of formulating the compound identified and obtained or a derivative thereof in a pharmaceutically acceptable form.
29. A method of diagnosing a disorder related to the presence of a molecular variant of an OCT1 gene or susceptibility to such a disorder comprising determining the presence of a polynucleotide of any one of item 1 to 3 or the gene of item 4 or 5 in a sample from a subject.
30. The method of item 29 further comprising determining the presence of a polypeptide of item 11 or the antibody of any one of item 12 to 14.
31. A method of diagnosing a disorder related to the presence of a molecular variant of an OCT1 gene or susceptibility to such a disorder comprising determining the presence of a polypeptide of item 11 or the antibody of any one of item 12 to 14 in a sample from a subject.
32. The method of any one of item 29 to 31, wherein said disorder comprises side effects, or reduced activity of drug therapy, or non-activity of drug therapy as a result from aberrant serum and/or intracellular concentrations of compounds that are substrates of the transporter OCT1.
33. The method of any one of item 29 to 32 comprising DNA sequencing, hybridisation techniques, PCR based assays, fluorescent dye and quenching agent-based PCR assay (Taqman PCR detection system), RFLP-based techniques, single strand conformational polymorphism (SSCP), denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, DHPLC-based techniques, oligonucleotide extension assays (OLA), extension based assays (ARMS, (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), pyrosequencing, protein truncation assay (PTT), immunoassays, haplotype analysis, and solid phase hydridization (dot blot, reverse dot blot, chips).
34. A method of detection of the polynucleotide of any one of item 1 to 3 or the gene of item 4 or 5 in a sample comprising the steps of
   (a) contacting the solid support of item 17 or 18 with the sample under conditions allowing interaction of the polynucleotide of item 1 to 3 or the gene of item 4 or 5 with the immobilized targets on a solid support and;
   (b) determining the binding of said polynucleotide or said gene to said immobilized targets on a solid support.
35. An in vitro method for diagnosing a disease comprising the steps of the method of item 34, wherein binding of said polynucleotide or gene to said immobilized targets on said solid support is indicative for the presence or the absence of said disease or a prevalence for said disease.
36. A diagnostic composition comprising the polynucleotide of any one of item 1 to 3, the gene of item 4 or 5, the vector of item 6 or 7, the polypeptide of item 11 or the antibody of any one of the item 12 to 14.
37. A pharmaceutical composition comprising the polynucleotide of any one of item 1 to 3, the gene of item 4 or 5, the vector of item 6 or 7, the polypeptide of item 11 or the antibody of any of the item 12 to 14.
38. Use of the polynucleotide of any one of item 1 to 3, the gene of item 4 or 5, the vector of item 6 or 7, the polypeptide of item 11*,* the polynucleotides having the polynucleotide sequences of SEQ ID NO: 18, 19, 20, 21, 22, 23, 24, 25, 26 or 27, the polypeptide of SEQ ID NO: 34 or 35, or the antibody of any of item 12 to 14 for the preparation of a diagnostic composition for diagnosing a disease.
39. Use of the polynucleotide of any one of item 1 to 3, the gene of item 4 or 5, the vector of item 6 or 7, the polypeptide of item 11, the polynucleotides having the polynucleotide sequences of SEQ ID NO: 18, 19, 20, 21, 22, 23, 24, 25, 26 or 27, the polypeptide of SEQ ID NO: 34 or 35, or the antibody of any of the item 12 to 14 for the preparation of a pharmaceutical composition for treating a disease.
40. The use of item 38 or 39, wherein said disease comprises side effects, or reduced activity, or non-activity of drug therapy as a result from aberrant serum and/or intracellular concentrations of compounds that are substrates of the transporter OCT1.
41. A diagnostic kit for detection of a single nucleotide polymorphism comprising the polynucleotide of any one of item 1 to 3, the gene of item 4 or 5, the vector of item 6 or 7, the polypeptide of item 11, the antibody of any of the item 12 to 14, the host cell of item 8, the transgenic non-human animal of item 15 or 16 or the solid support of item 17 or 18.

## Claims

1. A polynucleotide comprising a polynucleotide selected from the group consisting of:
(a) a polynucleotide having the nucleic acid sequence of SEQ ID NO: 8;
(b) a polynucleotide encoding a polypeptide having the amino acid sequence of SEQ ID NO: 30;
(c) a polynucleotide having a nucleic acid sequence with at least 70%, preferably at least 75%, at least 80%, at least 85%, at least 90% or at least 95% sequence identity to an OCT1 gene, wherein said polynucleotide is having a nucleotide exchange or a nucleotide deletion of at least one nucleotide at position 126806 of the OCT1 gene (GenBank Accession No: Gl : 9581607);
(d) a polynucleotide capable of hybridizing to an OCT1 gene, wherein said polynucleotide is having a substitution of at least one nucleotide at a position corresponding to position 126806 of the OCT1 gene (GenBank Accession No: Gl : 9581607);
(e) a polynucleotide capable of hybridizing to an OCT1 gene, wherein said polynucleotide is having an A at a position corresponding to position 126806 of the OCT1 gene (GenBank Accession No: Gl : 9581607);
(f) a polynucleotide encoding an OCT1 polypeptide or fragment thereof, wherein said polypeptide comprises an amino acid substitution at position 401 of the OCT1 polypeptide (GenBank Accession No: Gl : 2511670); and
(g) a polynucleotide encoding an OCT1 polypeptide or fragment thereof, wherein said polypeptide comprises an amino acid substitution of G to S at position 401 of the OCT1 polypeptide (GenBank Accession No: Gl : 2511670).

2. A polynucleotide comprising a polynucleotide selected from the group consisting of:
(a) a polynucleotide having the nucleic acid sequence of SEQ ID NO: 4, 5, 6, 7, 8, 9, 10, 11, 12, 13, 14, 15, 16 or 17;
(b) a polynucleotide encoding a polypeptide having the amino acid sequence of SEQ ID NO: 28, 29, 30, 31, 32 or 33;
(c) a polynucleotide having a nucleic acid sequence with at least 70%, preferably at least 75%, at least 80%, at least 85%, at least 90% or at least 95% sequence identity to an OCT1 gene, wherein said polynucleotide is having a nucleotide exchange or a nucleotide deletion of at least one nucleotide at a position 109130, 109211, 119220, 123551, 126806, 126846, 126863 to 126865, 126922, 126915, 130672, 141819, 142951, 141961 or 142993 of the OCT1 gene (GenBank Accession No: Gl : 9581607);
(d) a polynucleotide capable of hybridizing to an OCT1 gene, wherein said polynucleotide is having a substitution of at least one nucleotide at a position corresponding to position 109130,109211, 119220, 123551, 126806,126846, 126922, 126915, 130672, 141819, 142951, 141961 or 142993 of the OCT1 gene (GenBank Accession No: Gl : 9581607) or a deletion of three nucleotides at a position corresponding to position 126863 to 126865 of the OCT1 gene (GenBank Accession No: Gl : 9581607);
(e) a polynucleotide capable of hybridizing to an OCT1 gene, wherein said polynucleotide is having an A at a position corresponding to position 126806, 141819, 142951 or 142993 of the OCT1 gene (GenBank Accession No: Gl : 9581607), a C at a position corresponding to position 109211 or 126846 of the OCT1 gene (GenBank Accession No: Gl : 9581607), a G at a position corresponding to position 126922 or 130672 of the OCT1 gene (GenBank Accession No: Gl : 9581607), a T at a position corresponding to position 109130,119220, 123551, 126915 or 141961 of the OCT1 gene (GenBank Accession No: Gl : 9581607) or an ATG deletion at a position corresponding to position 126863 to 126865 of the OCT1 gene (GenBank Accession No: Gl : 9581607);
(f) a polynucleotide encoding an OCT1 polypeptide or fragment thereof, wherein said polypeptide comprises an amino acid substitution at position 61, 88, 401, 414 or 465 of the OCT1 polypeptide (GenBank Accession No: Gl : 2511670); and
(g) a polynucleotide encoding an OCT1 polypeptide or fragment thereof, wherein said polypeptide comprises an amino acid substitution of R to C at position 61, an amino acid substitution of C to R at position 88, an amino acid substitution of G to S at position 401, an amino acid substitution of G to A at position 414, an amino acid deletion of M at position 420 or an amino acid substitution of G to R at position 465 of the OCT1 polypeptide (GenBank Accession No: Gl : 2511670).

3. The polynucleotide of claim 1 or 2, wherein
(a) said polynucleotide is associated with side effects, or reduced activity of drug-therapy, or non-activity of drug therapy as a result from aberrant serum and/or intracellular concentrations of compounds that are substrates of the transporter OCT1; and/or
(b) said polynucleotide is DNA or RNA.

4. A gene comprising the polynucleotide of claim 1, 2, or 3, wherein optionally a nucleotide deletion and/or substitution results in altered expression of the variant gene compared to the corresponding wild type gene.

5. A vector comprising a polynucleotide of any one of claims 1 to 3 or the gene of claim 4; wherein the polynucleotide is optionally operatively linked to expression control sequences allowing expression in prokaryotic or eukaryotic cells or isolated fractions thereof.

6. A host cell genetically engineered with the polynucleotide of any one of claims 1 to 3, the gene of claim 4 or the vector of claim 5.

7. A method for producing
(a) a molecular variant OCT1 polypeptide or fragment thereof comprising
(i) culturing the host cell of claim 6; and
(ii) recovering said protein or fragment from the culture; or
(b) cells capable of expressing a molecular variant OCT1 polypeptide comprising genetically engineering cells with the polynucleotide of any one of claims 1 to 3, the gene of claim 4 or the vector of claim 5.

8. A polypeptide or fragment thereof encoded by the polynucleotide of any one of claims 1 to 3, the gene of claim 4 or obtainable by the method of claim 7(a) or from cells produced by the method of claim 7(b).

9. An antibody which binds specifically to the polypeptide of claim 8, wherein
(a) the antibody optionally specifically recognizes an epitope containing one or more amino acid substitution(s) resulting from a nucleotide exchange as defined in claim 1, 2 or 4; and/or
(b) the antibody is polyclonal or monoclonal.

10. A transgenic non-human animal comprising at least one polynucleotide of any one of claims 1 to 3, the gene of claim 4 or the vector of claim 5, wherein the transgenic non-human animal optionally is a mouse, a rat or a zebrafish.

11. A solid support comprising one or a plurality of the polynucleotide of any one of claims 1 to 3, the gene of claim 4, the vector of claim 5, the polypeptide of claim 8, the antibody of claim 9 or the host cell of claim 6 in immobilized form; wherein said solid support is optionally a membrane, a glass-or polypropylene-or silicon-chip, are oligonucleotide-conjugated beads or a bead array, which is assembled on an optical filter substrate.

12. An in vitro method for identifying a single nucleotide polymorphism said method comprising the steps of:
(a) isolating a polynucleotide of any one claims 1 to 3 or the gene of claim 4 from a plurality of subgroups of individuals, wherein one subgroup has no prevalence for an OCT1 associated disease and at least one or more further subgroup(s) do have prevalence for an OCT1 associated disease; and
(b) identifying a single nucleotide polymorphism by comparing the nucleic acid sequence of said polynucleotide or said gene of said one subgroup having no prevalence for an OCT1 associated disease with said at least one or more further subgroup(s) having a prevalence for an OCT1 associated disease.

13. A method for identifying and obtaining
(a) a pro-drug or a drug capable of modulating the activity of a molecular variant of an OCT1 polypeptide comprising the steps of:
(i) contacting the polypeptide of claim 8, the solid support of claim 11, a cell expressing a molecular variant gene comprising a polynucleotide of any one of claims 1 to 3, the gene of claim 4 or the vector of claim 5 in the presence of components capable of providing a detectable signal in response to drug activity with a compound to be screened for pro-drug or drug activity; and
(ii) detecting the presence or absence of a signal or increase or decrease of a signal generated from the pro-drug or the drug activity, wherein the absence, presence, increase or decrease of the signal is indicative for a putative pro-drug or drug; or
(b) an inhibitor of the activity of a molecular variant of an OCT1 polypeptide comprising the steps of:
(i) contacting the protein of claim 8, the solid support of claim 11 or a cell expressing a molecular variant gene comprising a polynucleotide of any one of claims 1 to 3 or the gene of claim 4 or the vector of claim 5 in the presence of components capable of providing a detectable signal in response to drug activity with a compound to be screened for inhibiting activity; and
(ii) detecting the presence or absence of a signal or increase or decrease of a signal generated from the inhibiting activity, wherein the absence or decrease of the signal is indicative for a putative inhibitor;
wherein said cell optionally is a cell of claim 6, obtained by the method of claim 7(b) or can be obtained by the transgenic non-human animal of claim 10.

14. A method of identifying and obtaining
(a) a pro-drug or drug capable of modulating the activity of a molecular variant of an OCT1 polypeptide comprising the steps of:
(i) contacting the host cell of claim 6, the cell obtained by the method of claim 7(b), the polypeptide of claim 8 or the solid support of claim 11 item with the first molecule known to be bound by an OCT1 polypeptide to form a first complex of said polypeptide and said first molecule;
(ii) contacting said first complex with a compound to be screened, and
(iii) measuring whether said compound displaces said first molecule from said first complex; or
(b) an inhibitor capable of modulating the activity of a molecular variant of an OCT1 polypeptide comprising the steps of:
(i) contacting the host cell of claim 6, the cell obtained by the method of claim 7(b), the protein of claim 8 or the solid support of claim 11 with the first molecule known to be bound by an OCT1 polypeptide to form a first complex of said polypeptide and said first molecule;
(ii) contacting said first complex with a compound to be screened, and
(iii) measuring whether said compound displaces said first molecule from said first complex;
wherein optionally
said measuring step comprises measuring the formation of a second complex of
said polypeptide and said compound; and/or
said measuring step comprises measuring the amount of said first molecule that is
not bound to said polypeptide; and/or
said first molecule is labeled.

15. A method for the production of a pharmaceutical composition comprising the steps of the method of claims 13 or 14; and the further step of formulating the compound identified and obtained or a derivative thereof in a pharmaceutical acceptable form.

16. A method of diagnosing a disorder related to the presence of a molecular variant of an OCT1 gene or susceptibility to such a disorder comprising
(a) determining the presence of a polynucleotide of any one of claims 1 to 3 or the gene of claim 4 in a sample from a subject, the method optionally further comprising determining the presence of a polypeptide of claim 8 or the antibody of claim 9; or
(b) determining the presence of a polypeptide of claim 8 or the antibody of claim 9 in a sample from a subject;
wherein optionally
said disorder comprises side effects, or reduced activity of drug therapy, or non-activity of drug therapy as a result from aberrant serum and/or intracellular concentrations of compounds that are substrates of the transporter OCT1; and/or the method comprises DNA sequencing, hybridisation techniques, PCR based assays, fluorescent dye and quenching agent-based PCR assay (Taqman PCR detection system), RFLP-based techniques, single strand conformational polymorphism (SSCP), denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, DHPLC-based techniques, oligonucleotide extension assays (OLA), extension based assays (ARMS, (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), pyrosequencing, protein truncation assay (PTT), immunoassays, haplotype analysis, and solid phase hydridization (dot blot, reverse dot blot, chips).

17. A method of detection of the polynucleotide of any one of claims 1 to 3 or the gene of claim 4 in a sample comprising the steps of
(a) contacting the solid support of claim 11 with the sample under conditions allowing interaction of the polynucleotide of claim 1 to 3 or the gene of claim 4 with the immobilized targets on a solid support and;
(b) determining the binding of said polynucleotide or said gene to said immobilized targets on a solid support.

18. An in vitro method for diagnosing a disease comprising the steps of the method of claim 17, wherein binding of said polynucleotide or gene to said immobilized targets on said solid support is indicative for the presence or the absence of said disease or a prevalence for said disease.

19. A pharmaceutical or diagnostic composition comprising the polynucleotide of any one of claims 1 to 3, the gene of claim 4, the vector of claim 5, the polypeptide of claim 8 or the antibody of claim 9.

20. The polynucleotide of any one of claims 1 to 3, the gene of claim 4, the vector of claim 5, the polypeptide of claim 8, the polynucleotides having the polynucleotide sequences of SEQ ID NO: 18, 19, 20, 21, 22, 23, 24, 25, 26 or 27, the polypeptide of SEQ ID NO: 35 or 36, or the antibody of claim 9 for use for diagnosing or treating a disease, wherein said disease optionally comprises side effects, or reduced activity, or non-activity of drug therapy as a result from aberrant serum and/or intracellular concentrations of compounds that are substrates of the transporter OCT1.

21. A diagnostic kit for detection of a single nucleotide polymorphism comprising the polynucleotide of any one of claims 1 to 3, the gene of claim 4, the vector of claim 5, the polypeptide of claim 8, the antibody of claim 9, the host cell of claim 6, the transgenic non-human animal of claim 10 or the solid support of claim 11.
